# EUROPEAN PATENT APPLICATION

(11) **EP 4 635 418 A1**
(43) Date of publication of application: **22.10.2025**
(21) Application number: 25165504.9
(22) Date of filing: 24.03.2025
(51) Int. Cl.: A61B 5/339, A61B 5/00

(54) **ELECTROCARDIOGRAPHY ACQUISITION METHOD AND DEVICE**

(30) Priority: 28.03.2024 CN 202410372603; 28.04.2024 CN 202410524886
(71) Applicant: Shenzhen Mindray Bio-Medical Electronics Co., Ltd., Shenzhen, Guangdong 518057 (CN)
(72) Inventor: JI, Baimiao, Shenzhen, 518057 (CN); DENG, Zhuomin, Shenzhen, 518057 (CN); TAN, Yawen, Shenzhen, 518057 (CN); XU, Jihao, Shenzhen, 518057 (CN); ZHANG, Tao, Shenzhen, 518057 (CN); YAO, Zejiang, Shenzhen, 518057 (CN)
(74) Representative: KIPA AB

(57) **Abstract**

This disclosure provides an electrocardiography acquisition method and apparatus. The method displays a position corresponding relationship between a first position and a second position of a predetermined electrocardiographic lead in multiple electrocardiographic leads, or an information entry which is capable of viewing said relationship. The first position indicates a position of the target object at which position the lead electrode of the predetermined electrocardiographic lead is connected while acquiring first electrocardiographic data, the second position indicates a position of the target object at which position the lead electrode of the predetermined electrocardiographic lead is to be connected, the position corresponding relationship guides a user to move the lead electrode of the predetermined electrocardiographic lead from the first position to the second position, so as to assist a user to connect electrocardiographic lead more accurately, reduce a probability of wrongly connecting a reused predetermined electrocardiographic lead, and improve use experience.

## Description

### CROSS-REFERENCE TO RELATED APPLICATIONS

This disclosure claims priority to Chinese application No. 202410524886.3, filed on April 28, 2024, to Chinese Patent Office; and Chinese application No. 202410372603.8, filed on March 28, 2024, to Chinese Patent Office; the contents of which are incorporated herein by reference in their entirety.

### TECHNICAL FIELD

The disclosure relates to a technical field of medical device, and more particularly to an electrocardiography acquisition method and an electrocardiography acquisition apparatus.

### BACKGROUND

In clinical practice, in order to satisfy a requirement of health examination for an object to be examined, such as a patient, electrocardiographic data acquisition, such as 12-lead electrocardiographic data acquisition, is often required. However, 12-lead electrocardiographic data can only cover anterior and inferior walls of myocardium, but cannot fully cover other positions of heart.

In order to obtain more electrocardiographic data, more leads are added on the basis of 12-lead electrocardiographic measurement, so as to obtain 15-lead electrocardiographic data or 18-lead electrocardiographic data. For example, when myocardial infarction of posterior wall is suspected, three additional leads of posterior wall need to be used for measurement. When a right ventricular disease is suspected, three additional leads of right chest should be used for measurement.

At present, a widely used electrocardiography acquisition apparatus, such as an ECG (electrocardiogram) device, is a 12-lead ECG device. When a multi-lead electrocardiographic measurement, which is not the 12-lead electrocardiographic measurement, needs to be implemented, only after the conventional 12-lead electrocardiographic measurement is completed, corresponding electrodes of conventional 12-lead electrocardiographic measurement can be reused to implement an additional leads operation.

However, operation guidance for the additional leads operation in related technologies is not clear. During performing an additional leads operation, an accuracy of such operation depends on a user with high professional operation skills, which makes a use experience of product have certain defects and is not conducive to product promotion.

### SUMMARY

A main purpose for an embodiment of this disclosure is to provide an electrocardiography acquisition method and an electrocardiography acquisition apparatus, aiming to assist a user to accurately and efficiently complete an additional leads operation, so as to improve a use experience of electrocardiography acquisition product.

In a first aspect, an embodiment of this disclosure provides an electrocardiography acquisition method, which is applied to an electrocardiography acquisition apparatus, including:
receiving a first acquisition instruction; and in response to the first acquisition instruction, obtaining first electrocardiographic data, which is acquired by lead electrode(s) of multiple electrocardiographic leads which are connected with a target object;
receiving an addition instruction; in response to the addition instruction, displaying, on a display interface of the electrocardiography acquisition apparatus, a position corresponding relationship between a first position and a second position of a predetermined electrocardiographic lead in the multiple electrocardiographic leads, or an information entry which is capable of viewing a position corresponding relationship between the first position and the second position; wherein the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receiving a second acquisition instruction; and in response to the second acquisition instruction, obtaining second electrocardiographic data which is acquired by the lead electrode(s) of the multiple electrocardiographic leads.

In a second aspect, an embodiment of this disclosure provides an electrocardiography acquisition method, which is applied to an electrocardiography acquisition apparatus, including:
determining historical electrocardiographic data of a target object;
receiving an addition instruction; in response to the addition instruction, displaying, on a display interface of the electrocardiography acquisition apparatus, a position corresponding relationship between a first position and a second position of a predetermined electrocardiographic lead in multiple electrocardiographic leads, or an information entry which is capable of viewing a position corresponding relationship between the first position and the second position; wherein the historical electrocardiographic data is data acquired by lead electrode(s) of the multiple electrocardiographic leads and obtained by the electrocardiography acquisition apparatus in response to a first acquisition instruction; wherein the first position is configured to indicate a position of the target object at which position a lead electrode of the predetermined electrocardiographic lead is connected while acquiring the historical electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receiving a second acquisition instruction; and in response to the second acquisition instruction, obtaining second electrocardiographic data which is acquired by the lead electrode(s) of the multiple electrocardiographic leads.

In a third aspect, an embodiment of this disclosure provides an electrocardiography acquisition method, which is applied to an electrocardiography acquisition apparatus, including:
receiving a first acquisition instruction; and in response to the first acquisition instruction, obtaining first electrocardiographic data, which is acquired by lead electrode(s) of multiple electrocardiographic leads which are connected with a target object;
receiving an addition instruction; in response to the addition instruction, displaying, on a display interface of the electrocardiography acquisition apparatus, a graphical position corresponding relationship between a first position and a second position of a predetermined electrocardiographic lead in the multiple electrocardiographic leads; or in response to the addition instruction, displaying, on a display interface of the electrocardiography acquisition apparatus, a graphical information entry which is capable of viewing a position corresponding relationship between a first position and a second position, and when the graphical information entry is scanned by an electronic device, displaying the position corresponding relationship on a display interface of the electronic device; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the first electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, obtaining second electrocardiographic data which is acquired by the lead electrode(s) of the multiple electrocardiographic leads.

In a fourth aspect, an embodiment of this disclosure provides an electrocardiography acquisition method, which is applied to an electrocardiography acquisition apparatus, including:
determining historical electrocardiographic data of a target object;
receiving an addition instruction; in response to the addition instruction, displaying, on a display interface of the electrocardiography acquisition apparatus, a graphical position corresponding relationship between a first position and a second position of a predetermined electrocardiographic lead in multiple electrocardiographic leads; or in response to the addition instruction, displaying, on a display interface of the electrocardiography acquisition apparatus, a graphical information entry which is capable of viewing a position corresponding relationship between a first position and a second position, and when the graphical information entry is scanned by an electronic device, displaying the position corresponding relationship on a display interface of the electronic device; wherein the historical electrocardiographic data is data acquired by lead electrode(s) of the multiple electrocardiographic leads and obtained by the electrocardiography acquisition apparatus in response to a first acquisition instruction; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the historical electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, obtaining second electrocardiographic data which is acquired by the lead electrode(s) of the multiple electrocardiographic leads.

In a fifth aspect, an embodiment of this disclosure provides an electrocardiography acquisition apparatus, including an electrocardiography acquisition host, and a control apparatus which is in communicative connection with the electrocardiography acquisition host; wherein the control apparatus is configured to:
receive a first acquisition instruction; and in response to the first acquisition instruction, control the electrocardiography acquisition host to obtain first electrocardiographic data, which is acquired by lead electrode(s) of multiple electrocardiographic leads which are connected with a target object;
receive an addition instruction; and in response to the addition instruction, control the electrocardiography acquisition host to display a position corresponding relationship between a first position and a second position of a predetermined electrocardiographic lead in the multiple electrocardiographic leads, or an information entry which is capable of viewing a position corresponding relationship between a first position and a second position; wherein the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receive a second acquisition instruction; and in response to the second acquisition instruction, control the electrocardiography acquisition host to obtain second electrocardiographic data which is acquired by the lead electrode(s) of the multiple electrocardiographic leads.

In a sixth aspect, an embodiment of this disclosure provides an electrocardiography acquisition apparatus, including an electrocardiography acquisition host, and a control apparatus which is in communicative connection with the electrocardiography acquisition host; wherein the control apparatus is configured to:
determine historical electrocardiographic data of a target object;
receive an addition instruction; and in response to the addition instruction, control the electrocardiography acquisition host to display a position corresponding relationship between a first position and a second position of a predetermined electrocardiographic lead in multiple electrocardiographic leads, or an information entry which is capable of viewing a position corresponding relationship between a first position and a second position; wherein the historical electrocardiographic data is data acquired by lead electrode(s) of the multiple electrocardiographic leads and obtained by the electrocardiography acquisition apparatus in response to a first acquisition instruction; wherein the first position is configured to indicate a position of the target object at which position a lead electrode of the predetermined electrocardiographic lead is connected while acquiring the historical electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receive a second acquisition instruction; and in response to the second acquisition instruction, control the electrocardiography acquisition host to obtain second electrocardiographic data which is acquired by the lead electrode(s) of the multiple electrocardiographic leads.

In a seventh aspect, an embodiment of this disclosure provides an electrocardiography acquisition apparatus, including an electrocardiography acquisition host, and a control apparatus which is in communicative connection with the electrocardiography acquisition host; wherein the control apparatus is configured to:
receive a first acquisition instruction; and in response to the first acquisition instruction, control the electrocardiography acquisition host to obtain first electrocardiographic data, which is acquired by lead electrode(s) of multiple electrocardiographic leads which are connected with a target object;
receive an addition instruction; in response to the addition instruction, control the electrocardiography acquisition host to display a graphical position corresponding relationship between a first position and a second position of a predetermined electrocardiographic lead in the multiple electrocardiographic leads; or in response to the addition instruction, control the electrocardiography acquisition host to display a graphical information entry which is capable of viewing a position corresponding relationship between a first position and a second position, and when the graphical information entry is scanned by an electronic device, display the position corresponding relationship on a display interface of the electronic device; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the first electrocardiographic data; the second position icon is located above the first image element, so as to indicate a position of the target object at which position the lead electrode of the predetermined electrocardiographic lead is to be connected;
receive a second acquisition instruction; and in response to the second acquisition instruction, control the electrocardiography acquisition host to obtain second electrocardiographic data which is acquired by the lead electrode(s) of the multiple electrocardiographic leads.

In an eight aspect, an embodiment of this disclosure provides an electrocardiography acquisition apparatus, including an electrocardiography acquisition host, and a control apparatus which is in communicative connection with the electrocardiography acquisition host; wherein the control apparatus is configured to:
determine historical electrocardiographic data of a target object;
receive an addition instruction; in response to the addition instruction, control the electrocardiography acquisition host to display a graphical position corresponding relationship between a first position and a second position of a predetermined electrocardiographic lead in the multiple electrocardiographic leads; or in response to the addition instruction, control the electrocardiography acquisition host to display a graphical information entry which is capable of viewing a position corresponding relationship between a first position and a second position, and when the graphical information entry is scanned by an electronic device, display the position corresponding relationship on a display interface of the electronic device; wherein the historical electrocardiographic data is data acquired by lead electrode(s) of the multiple electrocardiographic leads and obtained by the electrocardiography acquisition apparatus in response to a first acquisition instruction; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the historical electrocardiographic data; the second position icon is located above the first image element, so as to indicate a position of the target object at which position the lead electrode of the predetermined electrocardiographic lead is to be connected;
receive a second acquisition instruction; and in response to the second acquisition instruction, control the electrocardiography acquisition host to obtain second electrocardiographic data which is acquired by the lead electrode(s) of the multiple electrocardiographic leads.

Based on that a current general electrocardiography acquisition apparatus in the market is a 12-lead electrocardiography acquisition apparatus, when electrocardiographic data for more than 12 leads is necessary to be acquired, lead electrode(s), which correspond(s) to predetermined electrocardiographic lead(s) in the 12-lead electrocardiography, should be reused after completing the 12-lead electrocardiography acquisition, so as to realize 15-lead electrocardiographic data acquisition, or 18-lead electrocardiographic data acquisition.

It can be seen from the above technical solution provided by the disclosure that, after receiving the addition instruction, the electrocardiography acquisition apparatus of the disclosure displays, on a corresponding display interface, a graphical or graphic position corresponding relationship between a first position and a second position of a predetermined electrocardiographic lead in multiple electrocardiographic leads, or an information entry which is capable of viewing a position corresponding relationship between a first position and a second position; wherein the first position is configured to indicate a position of the target object at which position a lead electrode of the predetermined electrocardiographic lead is connected while acquiring first electrocardiographic data or historical electrocardiographic data; the second position is configured to indicate a position of the target object at which position the lead electrode of the predetermined electrocardiographic lead is to be connected; the position corresponding relationship is configured to guide a user to move the lead electrode of the predetermined electrocardiographic lead from the first position to the second position. **In** this way, during reusing the lead electrode of the predetermined electrocardiographic lead to acquire second electrocardiographic data, a corresponding user is given a relatively clear movement guidance for the predetermined electrocardiographic lead, so as to assist the user to connect the electrocardiographic lead with the target object for a second electrocardiography acquisition more accurately, that is, to assist the user to more accurately and efficiently complete an additional leads operation, so as to reduce a probability of connecting the reused predetermined electrocardiographic lead wrongly by the user, and improve a use experience of electrocardiography acquisition product.

It should be understood that the above general description and the following detailed description are only exemplary and explanatory, and do not limit the disclosure.

### BRIEF DESCRIPTION OF THE DRAWINGS

In order to more clearly explain the embodiments of this disclosure or the technical solutions in the prior art, the following briefly introduces the drawings which are needed to be used in the description of the embodiments or the prior art. It is obvious that the drawings in the following description are only some embodiments of this disclosure. For those skilled in the art, other drawings can be obtained from these accompanying drawings without paying any creative works.
FIG. 1 is a block diagram of an electrocardiography acquisition apparatus provided by an embodiment of this disclosure.
FIG. 2 is a flow chart for steps of an electrocardiography acquisition method provided by a first embodiment of the disclosure.
FIG. 3 is a diagram for positions at a target object, at which positions lead electrodes which correspond to multiple leads, are connected, during conventional electrocardiography acquisition.
FIGS. 4~6 are diagrams of positions at a target object, at which positions lead electrodes which correspond to multiple leads, are connected, during different types of additional electrocardiography acquisition.
FIG. 7 is a diagram for a report interface of 12-lead conventional electrocardiographic report.
FIG. 8 is a diagram for a report interface of 18-lead additional electrocardiographic report.
FIGS. 9~10 are diagrams showing changes of user interface for acquiring additional electrocardiographic data by triggering addition entries on different interfaces.
FIG. 11 is a flow chart for steps of an electrocardiography acquisition method provided by a second embodiment of the disclosure.
FIG. 12 is a flow chart for steps of an electrocardiography acquisition method provided by a third embodiment of the disclosure.
FIG. 13 is a flow chart for steps of an electrocardiography acquisition method provided by a fourth embodiment of the disclosure.

### DESCRIPTION OF THE PREFERRED EMBODIMENTS

The technical solutions in the embodiments of this disclosure will be described clearly and completely below in combination with the drawings in the embodiments of this disclosure. Obviously, the described embodiments are just some of the embodiments of this disclosure, not all of them. Based on the embodiments in this disclosure, all other embodiments obtained by those skilled in the art without making creative work fall within the protection scope of this disclosure.

In the description of this disclosure, unless otherwise specified and limited, terms, such as "assemble", "connection" and "connected with/to" should be understood in a broad sense, for example, a fixed connection, a connection which is capable of being disconnected, or an integral connection; or a mechanical connection or an electrical connection; a direct connection, or an indirect connection through an intermediate medium, or an internal connection between two components. For those skilled in the art, the specific meaning of the above terms in this disclosure can be understood in specific circumstances.

The flow chart shown in the attached drawing is only an example, not necessarily including all contents and operations/steps, nor must it be executed in the described order. For example, some operations/steps can also be decomposed, combined or partially merged, so the actual execution sequence may change according to the actual situation.

**In** clinical practice, in order to satisfy a requirement of health examination for an object to be examined, such as a patient, electrocardiographic data acquisition, such as 12-lead electrocardiographic data acquisition, is often required. However, 12-lead electrocardiographic data can only cover anterior and inferior walls of myocardium, but cannot fully cover other positions of heart.

**In** order to obtain more electrocardiographic data, more leads are added on the basis of 12-lead electrocardiographic measurement, so as to obtain 15-lead electrocardiographic data or 18-lead electrocardiographic data. For example, when myocardial infarction of posterior wall is suspected, three additional leads of posterior wall need to be used for measurement. When a right ventricular disease is suspected, three additional leads of right chest should be used for measurement.

Based on that a current general electrocardiography acquisition apparatus in the market is a 12-lead electrocardiography acquisition apparatus, when electrocardiographic data for more than 12 leads is necessary to be acquired, lead electrode(s), which correspond(s) to predetermined electrocardiographic lead(s) in the 12-lead electrocardiography, should be reused after completing the 12-lead electrocardiography acquisition, so as to realize 15-lead electrocardiographic data acquisition, or 18-lead electrocardiographic data acquisition.

During some lead electrode reusing, in order to accurately obtain corresponding electrocardiographic data, it is necessary to accurately adjust a relative position at a target object of a lead electrode of a predetermined electrocardiographic lead. However, operation guidance for additional leads operation during some lead electrode reusing in related technologies is not clear, resulting in that during performing an additional leads operation, an accuracy of such operation depends on a user with high professional operation skills, which makes a use experience of product have certain defects and is not conducive to product promotion.

Based on this, the disclosure provides an electrocardiography acquisition method and an electrocardiography acquisition apparatus, aiming to assist a user to accurately and efficiently complete an additional leads operation, so as to improve a use experience of electrocardiography acquisition product. The Electrocardiography acquisition method is applied to the electrocardiography acquisition apparatus.

Some embodiments of this disclosure are described in detail below in combination with the accompanying drawings. The following embodiments and features in the embodiments may be combined with each other without conflict.

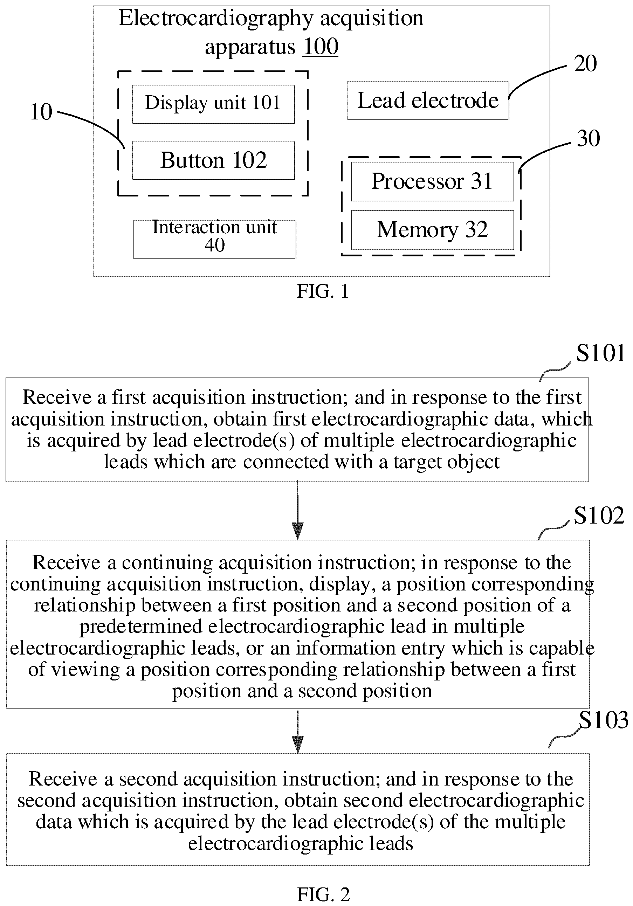

Please refer to FIG. 1, which is a block diagram of an electrocardiography acquisition apparatus provided by an embodiment of this disclosure.

As shown in FIG. 1, the electrocardiography acquisition apparatus 100 includes an electrocardiography acquisition host 10, a lead electrode 20, and a control apparatus 30. The lead electrode 20 is configured to contact a target object for acquiring electrocardiographic data. Multiple lead electrode 20 exists, and different lead electrodes are in contact with different positions of the target object. The electrocardiography acquisition host 10 is connected with the lead electrode 20, and is configured to acquire electrocardiographic data of the target object through the lead electrode 20, and the target object is a person to be implemented with electrocardiographic detection.

The control apparatus 30 is in communicative connection with the electrocardiography acquisition host 10, and is configured control the electrocardiography acquisition host 10 to perform a preset operation in response to a control instruction. For example, the electrocardiography acquisition host 10 is equipped with a display unit 101. The control apparatus 30 controls the electrocardiography acquisition host 10 to acquire the electrocardiographic data of the target object, and displays an electrocardiography waveform which corresponds to the electrocardiographic data of the target object, on a display interface which corresponds to the display unit 101; or based on an external control instruction, controls the electrocardiography acquisition host 10 to adjust a display effect of a electrocardiography waveform which corresponds to the acquired electrocardiographic data.

It can be understood that the control apparatus 30 can be one or more, and the control apparatus 30 can be arranged in at least one of the electrocardiography acquisition host 10 and the lead electrode 20, or can be independently arranged and in communicative connection with the electrocardiography acquisition host 10, which is not limited here.

In some embodiments, the electrocardiography acquisition apparatus 100 also includes an interaction unit 40, which is configured to receive an interaction instruction which is inputted by an external object, so as to provide a window for human-machine interaction between a user and the electrocardiography acquisition host 10. Wherein, the interaction unit 40 can be independently arranged relative to the electrocardiography acquisition host 10 and in communicative connection with the electrocardiography acquisition host 10, or can be arranged at the electrocardiography acquisition host 10 and in communicative connection with the control apparatus 30. There is no restriction here, and the external object includes but is not limited to a user.

Alternatively, the interaction unit 40 may be a physical key or a touch-control element which is arranged at the electrocardiography acquisition host 10, or a remote controller which is in communicative connection with the electrocardiography acquisition host 10, or a predetermined area at the display unit 101, or a virtual key or virtual icon which is arranged on the display interface which corresponds to the display unit 101.

In some embodiments, the control apparatus 30 at least includes a processor 31, a memory 32, a communication interface (not shown) and an I/O interface (not shown). The processor 31, the memory 32, the communication interface, and the I/O interface communicate with each other through a bus. The processor 31 can be a central processing unit (CPU) or another general-purpose processor, a digital signal processors (DSP), an application specific integrated circuit (ASIC), a field programmable gate array (FPGA) or another programmable logic device, a discrete gate, or a transistor logic device, a discrete hardware element, etc. The general-purpose processor can be a microprocessor or any conventional processor.

The memory 32 is loaded with various computer programs, such as an operation system, an application program for the processor 31 and so on, and data required to execute said programs, so as to execute said programs. During a defibrillation operation, if there is any data that needs to be stored locally, said data can be stored in the memory 32. The I/O interface includes but is not limited to, a serial interface, such as USB, IEEE1394 or RS-232C, a parallel interface, such as SCSI, IDE or IEEE1284, and an analog signal interface, which is composed of a D/A converter and a converter. An input component is connected with the I/O interface, and the user can directly input data to the control apparatus 30 with the input component, which component includes but is not limited to a keyboard, a mouse, a touch screen or a control button. The display component can be in communicative connection with the control apparatus 30 through the I/O interface, so as to implement relevant information indication. The communication interface can be an interface of any known communication protocol. The communication interface is in communicative connection with outside world through a network. Data transmission between the control apparatus 30 and any component which is connected through the network, can be performed by the control apparatus 30 through a communication interface with a preset communication protocol.

**In** some embodiments, the control apparatus 30 is configured to implement following method steps, specifically, the processor 31 of the control apparatus 30 invokes a computer program in the memory 32 to implement following method steps:
receiving a first acquisition instruction; and in response to the first acquisition instruction, controlling the electrocardiography acquisition host to obtain first electrocardiographic data, which is acquired by lead electrode(s) of multiple electrocardiographic leads which are connected with a target object;
receiving an addition instruction; and in response to the addition instruction, controlling the electrocardiography acquisition host 10 to display a position corresponding relationship between a first position and a second position of a predetermined electrocardiographic lead in the multiple electrocardiographic leads; in response to the addition instruction, controlling the electrocardiography acquisition host 10 to display an information entry which is capable of viewing a position corresponding relationship between a first position and a second position; wherein the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receiving a second acquisition instruction; and in response to the second acquisition instruction, controlling the electrocardiography acquisition host to obtain second electrocardiographic data which is acquired by the lead electrode(s) of the multiple electrocardiographic leads.

It can be understood that the first electrocardiographic data is conventional electrocardiographic data, and the second electrocardiographic data is additional electrocardiographic data.

In some embodiments, the control apparatus 30 is further configured to implement following method steps:
determining historical electrocardiographic data of a target object;
receiving an addition instruction; and in response to the addition instruction, controlling the electrocardiography acquisition host 10 to display a position corresponding relationship between a first position and a second position of a predetermined electrocardiographic lead in multiple electrocardiographic leads, or an information entry which is capable of viewing a position corresponding relationship between a first position and a second position; wherein first electrocardiographic data among the historical electrocardiographic data is data acquired by lead electrode(s) of the multiple electrocardiographic leads and obtained by the electrocardiography acquisition apparatus in response to a first acquisition instruction; wherein the first position is configured to indicate a position of the target object at which position a lead electrode of the predetermined electrocardiographic lead is connected while acquiring the historical electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receiving a second acquisition instruction; and in response to the second acquisition instruction, controlling the electrocardiography acquisition host 10 to obtain second electrocardiographic data which is acquired by the lead electrode(s) of the multiple electrocardiographic leads.

It can be understood that the historical electrocardiographic data is conventional electrocardiographic data which is acquired in the history, and the second electrocardiographic data is additional electrocardiographic data.

It can also be understood that, during determining historical electrocardiographic data of a target object, the control apparatus 30 is further configured to implement a following method step:
receiving a data selection instruction which is generated through triggering the electrocardiography acquisition apparatus by a user, and determining the historical electrocardiographic data of the target object according to the data selection instruction.

In some embodiments, the control apparatus 30 is further configured to implement following method steps:
acquiring identity information of a target object on whom an electrocardiographic detection is to be implemented;
receiving an addition instruction; in response to the addition instruction, control the electrocardiography acquisition host 10 to display a position corresponding relationship between a first position and a second position of a predetermined electrocardiographic lead in multiple electrocardiographic leads, or an information entry which is capable of viewing a position corresponding relationship between a first position and a second position; wherein the first position is configured to indicate an initial connection position between a lead electrode of the predetermined electrocardiographic lead of the electrocardiography acquisition apparatus and the target object, the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position;
receiving a second acquisition instruction; and in response to the second acquisition instruction, controlling the electrocardiography acquisition host 10 to obtain second electrocardiographic data which is acquired by lead electrode(s) of the multiple electrocardiographic leads.

In some embodiments, the control apparatus 30 is further configured to implement following method steps:
receiving a first acquisition instruction; and in response to the first acquisition instruction, controlling the electrocardiography acquisition host 10 to obtain first electrocardiographic data, which is acquired by lead electrode(s) of multiple electrocardiographic leads which are connected with a target object;
receiving an addition instruction; in response to the addition instruction, controlling the electrocardiography acquisition host 10 to display a graphical position corresponding relationship between a first position and a second position of a predetermined electrocardiographic lead in the multiple electrocardiographic leads; or in response to the addition instruction, controlling the electrocardiography acquisition host 10 to display a graphical information entry which is capable of viewing a position corresponding relationship between a first position and a second position, and when the graphical information entry is scanned by an electronic device, displaying the position corresponding relationship on a display interface of the electronic device; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the first electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, controlling the electrocardiography acquisition host 10 to obtain second electrocardiographic data which is acquired by the lead electrode(s) of the multiple electrocardiographic leads.

It can be understood that, the first electrocardiographic data is conventional electrocardiographic data, and the second electrocardiographic data is additional electrocardiographic data.

**In** some embodiments, the control apparatus 30 is further configured to implement following method steps:
determining historical electrocardiographic data of a target object;
receiving an addition instruction; in response to the addition instruction, controlling the electrocardiography acquisition host 10 to display a graphical position corresponding relationship between a first position and a second position of a predetermined electrocardiographic lead in the multiple electrocardiographic leads; or in response to the addition instruction, controlling the electrocardiography acquisition host 10 to display a graphical information entry which is capable of viewing a position corresponding relationship between a first position and a second position, and when the graphical information entry is scanned by an electronic device, displaying the position corresponding relationship on a display interface of the electronic device; wherein the historical electrocardiographic data is data acquired by lead electrode(s) of the multiple electrocardiographic leads and obtained by the electrocardiography acquisition apparatus in response to a first acquisition instruction; wherein the position corresponding relationship is configured to guide a user to move a lead electrode of the predetermined electrocardiographic lead from the first position to the second position; the position corresponding relationship at least includes a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the historical electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, controlling the electrocardiography acquisition host 10 to obtain second electrocardiographic data which is acquired by the lead electrode(s) of the multiple electrocardiographic leads.

It can be understood that the historical electrocardiographic data is conventional electrocardiographic data which is acquired in the history, and the second electrocardiographic data is additional electrocardiographic data.

It can also be understood that, during determining historical electrocardiographic data of a target object, the control apparatus 30 is further configured to implement a following method step:
receiving a data selection instruction which is generated through triggering the electrocardiography acquisition apparatus by a user, and determining the historical electrocardiographic data of the target object according to the data selection instruction.

It should be noted that those skilled in the art can clearly understand that for the convenience and simplicity of description, a specific working process of the steps of the electrocardiography acquisition method realized by the above described control apparatus can refer to corresponding working process of the electrocardiography acquisition method, and is not repeated here.

Please refer to FIG. 2, which is a flow chart for steps of an electrocardiography acquisition method provided by a first embodiment of the disclosure. The Electrocardiography acquisition method is applied to the aforementioned electrocardiography acquisition apparatus 100. The electrocardiography acquisition apparatus includes but is not limited to an electrocardiograph (ECG) machine. The following explains the electrocardiography acquisition method provided by an embodiment of this disclosure in combination with the working principle of the electrocardiography acquisition apparatus 100.

As shown in FIG. 2, the electrocardiography acquisition method includes steps S101 to S103.

Step S101: receive a first acquisition instruction; and in response to the first acquisition instruction, obtain first electrocardiographic data, which is acquired by lead electrode(s) of multiple electrocardiographic leads which are connected with a target object.

Step S102: receive a continuing acquisition instruction; and in response to the continuing acquisition instruction, display a position corresponding relationship between a first position and a second position of a predetermined electrocardiographic lead in the multiple electrocardiographic leads, or an information entry which is capable of viewing a position corresponding relationship between a first position and a second position; wherein the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position.

Step S103: receive a second acquisition instruction; and in response to the second acquisition instruction, obtain second electrocardiographic data which is acquired by the lead electrode(s) of the multiple electrocardiographic leads.

For example, the electrocardiographic lead include a limb lead and a chest lead, wherein the limb lead includes lead I, lead II, lead III, lead aVR, lead aVL, and lead aVF; the chest lead includes six precordial leads, and a back lead, that is, including precordial leads V1-V6; a lead V7, a lead V8 and a lead V9 on the left posterior chest wall; a lead V3R, a lead V4R and a lead V5R at the right chest.

A type of lead for electrocardiographic data include 12-lead, 15-lead and 18-lead. Usually, during implementing an electrocardiographic detection for a target object (such as a patient), medical staff can usually obtain 12-lead electrocardiographic data of the target object first, and make a preliminary diagnosis according to the 12-lead electrocardiographic data.

When suspecting that miscellaneous diseases, such as left ventricular hypertrophy or posterior myocardial infarction, exist in the left ventricle or the posterior wall of heart, obtained 12-lead electrocardiographic data is difficult to confirm such diagnosis, and multiple leads should be added on the basis of 12-lead measurement, so as to obtain 15-lead electrocardiographic data or 18-lead electrocardiographic data. For example, when suspecting that posterior myocardial infarction exists, three additional leads on the posterior wall is needed; when suspecting that a right ventricular disease exists, three additional leads on the right chest is needed.

Wherein, the conventional 12-lead includes limb leads (lead I, lead II, lead III**,** lead aVR, lead aVL, and lead aVF) and precordial leads V1-V6.

The 15-lead includes limb leads, and a lead V7, a lead V8 and a lead V9 on the left posterior chest wall; or the 15-lead includes limb leads, and a lead V3R, a lead V4R and a lead V5R at the right chest.

The 18-lead includes limb leads; a lead V7, a lead V8 and a lead V9 on the left posterior chest wall; and a lead V3R, a lead V4R and a lead V5R at the right chest.

The lead V3R, the lead V4R and the lead V5R at the right chest are mainly symmetrically placed with corresponding leads at the left chest. The lead V7, the lead V8 and the lead V9 on the left posterior chest wall are usually placed along a posterior axillary line of the fifth intercostal space on the left side of the human sternum, and along a scapular line and a paraspinal line.

Since most electrocardiography acquisition apparatus are not equipped with more lead electrodes than required for conventional lead detection (such as 12-lead), if leads for left posterior chest or right chest need to be added, the user needs to manually adjust corresponding lead electrodes, reuse electrodes which correspond to the conventional leads for additional detection, so as to obtain 15-lead electrocardiographic data or 18-lead electrocardiographic data.

In addition, the reusing of electrodes during an additional leads operation should satisfy following requirements: an electrode which corresponds to lead V1 is reused as an electrode of lead V3R, an electrode which corresponds to lead V2 is reused as an electrode of lead V4R, an electrode which corresponds to lead V3 is reused as an electrode of lead V5R, an electrode which corresponds to lead V4 is reused as an electrode of lead V9, an electrode which corresponds to lead V5 is reused as an electrode of lead V8, and an electrode which corresponds to lead V6 is reused as an electrode of lead V7.

If a reusing sequence of each lead is wrong, the electrocardiographic data obtained by each lead will be wrong, and an electrocardiographic report which is outputted according to the electrocardiographic data will be wrong. Therefore, the accuracy of the additional leads operation depends on a user with high professional operation skills.

In an embodiment of this disclosure, the first acquisition instruction is a conventional acquisition instruction, and the first electrocardiographic data is conventional electrocardiographic data, that is, the conventional acquisition instruction is used to control the electrocardiography acquisition apparatus 100 to acquire the conventional electrocardiographic data of the target object, such as 12-lead electrocardiographic data.

The second acquisition instruction is additional acquisition instruction, and the second electrocardiographic data is the additional electrocardiographic data, that is, the additional acquisition instruction is used to control the electrocardiography acquisition apparatus to acquire the additional electrocardiographic data of the target object, such as data for lead V7, lead V8, lead V9 on the left posterior chest wall, and/or lead V3R, lead V4R, lead V5R at the right chest. The 15-lead electrocardiographic data or 18-lead electrocardiographic data of the target object can be obtained by respectively acquiring the conventional electrocardiographic data and the additional electrocardiographic data of the target object.

While acquiring 12-lead electrocardiographic data, a user selects a 12-lead option at the electrocardiography acquisition apparatus 100, and then connects multiple lead electrodes 20 at preset positions of human body.

The 12-lead includes limb leads and six precordial leads. Limb leads include lead I**,** lead II**,** lead III**,** lead aVR, lead aVL, and lead aVF. Corresponding four lead electrodes 20 are further required to connect at preset portions of limbs of the target object.

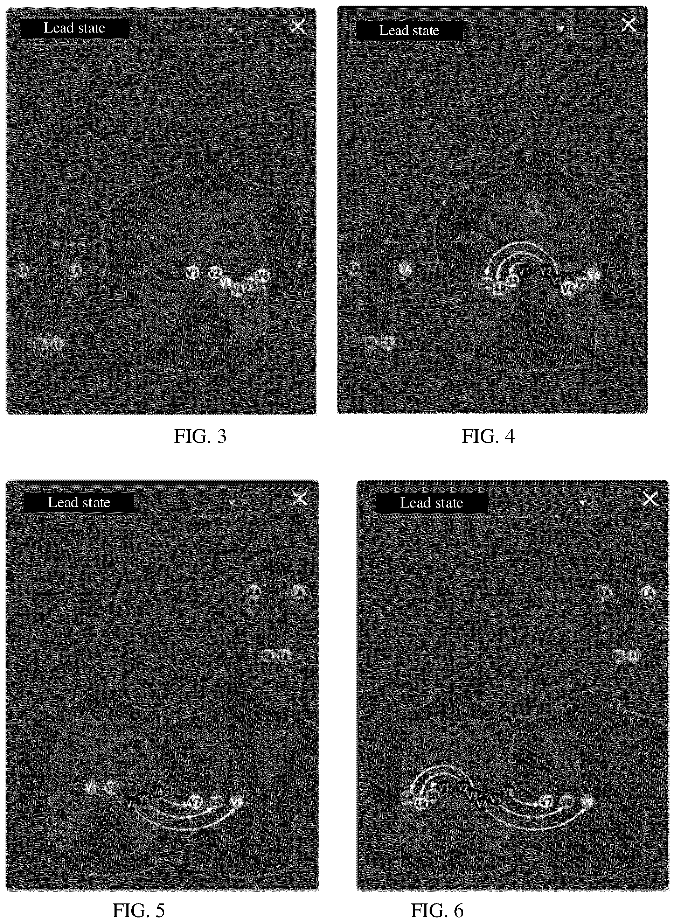

As shown in FIG. 3, an RA electrode in the multiple lead electrodes 20 is connected at the right upper limb of the target object, an LA electrode in the multiple lead electrodes 20 is connected at the left upper limb of the target object, an LL electrode in the multiple lead electrodes 20 is connected at the left lower limb of the target object, and an RL electrode in the multiple lead electrodes 20 is connected at the right lower limb of the target object. Limb lead data of the target object is obtained through the four lead electrodes.

The six precordial leads are leads V1-V6, and six lead electrodes 20 are required to be connected at six corresponding preset positions at the front chest of the target object. As shown in FIG. 3, an electrode which corresponds to lead V1 in multiple lead electrodes 20 is connected at a position V1 of a fourth intercostal space on a right side of human sternum, an electrode which corresponds to lead V2 is connected at a position V2 of a fourth intercostal space on a left side of human sternum, an electrode which corresponds to lead V4 is connected at a position V4 of a midclavicular line of a fifth intercostal space on a left side of human sternum, and an electrode which corresponds to lead V3 is connected at a position V3 between leads V2 and V4, an electrode which corresponds to lead V5 is connected at a position V5 at a point of intersection between a fifth intercostal space of human sternum and an anterior axillary line, and an electrode which corresponds to lead V6 is connected at a point of intersection between a fifth intercostal space of human sternum and a midaxillary line.

After connecting the lead electrodes 20, which correspond to the 12-lead electrocardiographic data to be acquired, at corresponding portions of the target object, the user transmits a first acquisition instruction to the electrocardiography acquisition apparatus 100 by triggering a first electrocardiography acquisition entry which is set by the interaction unit 40, so as to enable the electrocardiography acquisition apparatus 100 to acquire, in response to the electrocardiography acquisition instruction, first electrocardiographic data of the target object, that is, 12-lead electrocardiographic data of the target object, that is, electrocardiographic data which corresponds to lead I, lead II, lead III**,** lead aVR, lead aVL, lead aVF and leads V1-V6, also known as conventional electrocardiographic data or standard 12-lead data.

After completing the acquisition of the first electrocardiographic data, if additional leads data of the target object is further to be acquired, a corresponding continuing acquisition instruction is transmitted to the electrocardiography acquisition apparatus 100, by triggering a second electrocardiography acquisition entry which is set by the interaction unit 40, so as to enable the electrocardiography acquisition apparatus 100 to display, in response to the continuing acquisition instruction, on a display interface of the display unit 101 of the electrocardiography acquisition apparatus 100, an adjustment guidance for electrode position, which guidance corresponds to the continuing acquisition instruction. Wherein, the adjustment guidance for electrode position is a graphical adjustment guidance for electrode position. The adjustment guidance for electrode position includes a position corresponding relationship between a first position and a second position of a predetermined electrocardiographic lead in multiple electrocardiographic leads. Optional, the electrocardiography acquisition apparatus 100 displays, in response to the continuing acquisition instruction, on a display interface of the display unit 101 of the electrocardiography acquisition apparatus 100, an information entry which is capable of viewing a position corresponding relationship between a first position and a second position. Wherein, the predetermined electrocardiographic lead is a lead whose position is needed to be adjusted, the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position.

In an embodiment of this disclosure, the continuing acquisition instruction is used to trigger the electrocardiography acquisition apparatus 100 to display a graphical adjustment guidance for electrode position, so as to enable the user to perform the additional leads operation according to the adjustment guidance for electrode position. Therefore, the continuing acquisition instruction is also called as an addition instruction. For ease of understanding, the continuing acquisition instruction is hereinafter uniformly referred to as the addition instruction. Refer to FIG. 4. Take the additional leads operation on the right chest as an example. If the user wants to perform the additional leads operation on the right chest for the target object, the user transmits a corresponding first addition instruction (for example, an addition instruction for right chest lead) to the electrocardiography acquisition apparatus 100 through the interaction unit 40. The electrocardiography acquisition apparatus 100 displays a graphical first adjustment guidance for electrode position, according to the received first addition instruction. The first adjustment guidance for electrode position shows that leads, whose electrode position needs to be adjusted for the additional leads operation on the right chest, include lead V1, lead V2, lead V3. A lead, whose electrode position needs to be adjusted, is called as a predetermined electrocardiographic lead.

That is, a lead electrode to be adjusted is a lead electrode which corresponds to lead V1, a lead electrode which corresponds to lead V2, and a lead electrode which corresponds to lead V3. In addition, the reusing of the corresponding lead electrode should follow following rules: the electrode which corresponds to lead V1 is reused as an electrode of lead V3R, the electrode which corresponds to lead V2 is reused as an electrode of lead V4R, and the electrode which corresponds to lead V3 is reused as an electrode of lead V5R.

Therefore, the electrocardiography acquisition apparatus 100 displays on the corresponding display interface, first positions of the target object at which positions the predetermined electrocardiographic lead V1, lead V2, lead V3 are connected while acquiring conventional electrocardiography, and second positions of the human body at which positions the electrodes, which correspond to the predetermined electrocardiographic leads, are to be connected while needing to acquire additional leads data, wherein the second positions may include a position 3R which corresponds to the right chest lead V3R, a position 4R which corresponds to the right chest lead V4R, and a position 5R which corresponds to the right chest lead V5R.

At the same time, the electrocardiography acquisition apparatus 100 displays that: the electrode which corresponds to lead V1 needs to be moved to the position 3R which corresponds to lead V3R, so as to be used as the electrode of lead V3R; the electrode which corresponds to lead V2 needs to be moved to the position 4R which corresponds to lead V4R, so as to be used as the electrode of lead V4R; and the electrode which corresponds to lead V3 needs to be moved to the position 5R which corresponds to lead V5R, so as to be used as the electrode of lead V5R, thus giving the user a clear and accurate adjustment guidance for electrode position, enabling the user to accurately and efficiently adjust the electrode position according to the displayed adjustment guidance for electrode position.

After the user completes the electrode adjustment according to the adjustment guidance for electrode position, an additional electrocardiographic data acquisition can be performed. The user transmits a corresponding second acquisition instruction to the electrocardiography acquisition apparatus 100 through the interactive unit 40, and then controls, through the second acquisition instruction, the lead electrode of the multiple electrocardiographic leads, which electrode are connected with the target object, to acquire second electrocardiographic data of the target object. That is, at this time, the second electrocardiographic data is 15-lead electrocardiographic data of right chest.

Refer to FIG. 5. Take an additional leads operation on the left posterior chest wall as an example. If the user wants to perform the additional leads operation on the left posterior chest wall for the target object, the user transmits a corresponding second addition instruction (for example, an addition instruction for left posterior chest wall lead) to the electrocardiography acquisition apparatus 100 through the interaction unit 40. The electrocardiography acquisition apparatus 100 displays a second adjustment guidance for electrode position, according to the received second addition instruction. The second adjustment guidance for electrode position shows that the predetermined electrocardiographic leads, whose electrode position needs to be adjusted for the additional leads operation on the left posterior chest wall, include lead V4, lead V5, and lead V6.

That is, a lead electrode to be adjusted is a lead electrode which corresponds to lead V4, a lead electrode which corresponds to lead V5, and a lead electrode which corresponds to lead V6. In addition, the reusing of the corresponding lead electrode should follow following rules: the electrode which corresponds to lead V4 is reused as an electrode of lead V9, the electrode which corresponds to lead V5 is reused as an electrode of lead V8, and the electrode which corresponds to lead V6 is reused as an electrode of lead V7.

Therefore, the electrocardiography acquisition apparatus 100 displays on the corresponding display interface, first positions at the target object, at which positions the predetermined electrocardiographic lead V4, lead V5, lead V6 are connected while acquiring conventional electrocardiography, and second positions of the human body at which positions the electrodes, which correspond to the predetermined electrocardiographic lead, are to be connected, while needing to acquire additional leads data, wherein the second positions may include a position V7 which corresponds to the left posterior chest wall lead V7, a position V8 which corresponds to the left posterior chest wall lead V8, and a position V9 which corresponds to the left posterior chest wall lead V9.

At the same time, the electrocardiography acquisition apparatus 100 displays that: the electrode which corresponds to lead V4 needs to be moved to the position V9 which corresponds to lead V9, so as to be used as the electrode of lead V9; the electrode which corresponds to lead V5 needs to be moved to the position V8 which corresponds to lead V8, so as to be used as the electrode of lead V8; and the electrode which corresponds to lead V6 needs to be moved to the position V7 which corresponds to lead V7, so as to be used as the electrode of lead V7, thus giving the user a clear and accurate adjustment guidance for electrode position, enabling the user to accurately and efficiently adjust the electrode position according to the displayed adjustment guidance for electrode position.

After the user completes the electrode adjustment according to the adjustment guidance for electrode position, an additional electrocardiographic data acquisition can be performed. The user transmits a corresponding second acquisition instruction to the electrocardiography acquisition apparatus 100 through the interactive unit 40, and then controls, through the second acquisition instruction, the lead electrode of the multiple electrocardiographic leads, which electrode are connected with the target object, to acquire second electrocardiographic data of the target object. That is, at this time, the second electrocardiographic data is 15-lead electrocardiographic data of left posterior chest wall.

Refer to FIG. 6. Similarly, take an additional leads operation on the right chest and the left posterior chest wall as an example. If the user wants to perform the additional leads operation on the right chest and the left posterior chest for the target object, the user transmits a corresponding third addition instruction (for example, an addition instruction for right chest lead and left posterior chest wall lead) to the electrocardiography acquisition apparatus 100 through the interaction unit 40. The electrocardiography acquisition apparatus 100 displays the third adjustment guidance for electrode position, according to the received third addition instruction. The third adjustment guidance for electrode position shows that leads, whose electrode position needs to be adjusted for the additional leads operation on the right chest and the left posterior chest wall, include lead V1, lead V2, lead V3, lead V4, lead V5, and lead V6. A lead, whose electrode position needs to be adjusted, is called as a predetermined electrocardiographic lead.

That is, a lead electrode to be adjusted is a lead electrode which corresponds to lead V1, a lead electrode which corresponds to lead V2, a lead electrode which corresponds to lead V3, a lead electrode to be adjusted is a lead electrode which corresponds to lead V4, a lead electrode which corresponds to lead V5, and a lead electrode which corresponds to lead V6.

In addition, the reusing of the corresponding lead electrode should follow following rules: the electrode which corresponds to lead V1 is reused as an electrode of lead V3R, the electrode which corresponds to lead V2 is reused as an electrode of lead V4R, and the electrode which corresponds to lead V3 is reused as an electrode of lead V5R, the electrode which corresponds to lead V4 is reused as an electrode of lead V9, the electrode which corresponds to lead V5 is reused as an electrode of lead V8, and the electrode which corresponds to lead V6 is reused as an electrode of lead V7.

Therefore, the electrocardiography acquisition apparatus 100 displays on the corresponding display interface, first positions of the target object, at which positions the predetermined electrocardiographic lead V1, lead V2, and lead V3, lead V4, lead V5, and lead V6 are connected while acquiring conventional electrocardiography; and second positions of the human body at which positions the electrodes, which correspond to the predetermined electrocardiographic lead, are to be connected, while needing to acquire additional leads data, wherein the second positions may include a position 3R which corresponds to the right chest lead V3R, a position 4R which corresponds to the right chest lead V4R, a position 5R which corresponds to the right chest lead V5R, a position V7 which corresponds to the left posterior chest wall lead V7, a position V8 which corresponds to the left posterior chest wall lead V8, and a position V9 which corresponds to the left posterior chest wall lead V9.

At the same time, the electrocardiography acquisition apparatus 100 displays that: the electrode which corresponds to lead V1 needs to be moved to the position 3R which corresponds to lead V3R, so as to be used as the electrode of lead V3R; the electrode which corresponds to lead V2 needs to be moved to the position 4R which corresponds to lead V4R, so as to be used as the electrode of lead V4R; the electrode which corresponds to lead V3 needs to be moved to the position 5R which corresponds to lead V5R, so as to be used as the electrode of lead V5R; the electrode which corresponds to lead V4 needs to be moved to the position V9 which corresponds to lead V9, so as to be used as the electrode of lead V9; the electrode which corresponds to lead V5 needs to be moved to the position V8 which corresponds to lead V8, so as to be used as the electrode of lead V8; and the electrode which corresponds to lead V6 needs to be moved to the position V7 which corresponds to lead V7, so as to be used as the electrode of lead V7, thus giving the user a clear and accurate adjustment guidance for electrode position, enabling the user to accurately and efficiently adjust the electrode position according to the displayed adjustment guidance for electrode position.

After the user completes the electrode adjustment according to the adjustment guidance for electrode position, an additional electrocardiographic data acquisition can be performed. The user transmits a corresponding second acquisition instruction to the electrocardiography acquisition apparatus 100 through the interactive unit 40, and then controls, through the second acquisition instruction, the lead electrode of the multiple electrocardiographic leads, which electrode are connected with the target object, to acquire second electrocardiographic data of the target object. That is, at this time, the second electrocardiographic data is 18-lead electrocardiographic data of right chest and left posterior chest wall.

In some embodiments, the position corresponding relationship of the predetermined electrocardiographic lead at least includes a first image element, a first position icon which corresponds to the first position, a second position icon which corresponds to the second position, and movement guidance information which indicates to move the lead electrode of the predetermined electrocardiographic lead between the first position and the second position. Wherein, the first image element is configured to simulate a target object and/or region(s) at the target object which region(s) is(are) connected with lead electrode(s) of multiple electrocardiographic leads, and the first position icon is located above the first image element, so as to indicate a position of the target object at which position a lead electrode of the predetermined electrocardiographic lead is connected, during acquiring first electrocardiographic data; the second position icon is located above the first image element, so as to indicate a position of the target object at which position the lead electrode of the predetermined electrocardiographic lead is to be connected, during acquiring second electrocardiographic data. Wherein one or more first image elements exist, optionally, two or three first image elements exist, and respective positions of the target object which are respectively indicated by each first image element are different, for example, limbs of the target object are indicated by one first image element, back of the target object is indicated by one first image element, and front chest of the target object indicated by one first image element.

Based on the position corresponding relationship of the predetermined electrocardiographic lead, a position relationship between lead electrodes of the predetermined electrocardiographic lead is indicated by the image element. Therefore, the position corresponding relationship of the predetermined electrocardiographic leads is a graphical position corresponding relationship.

Alternatively, after the user scans the information entry which is displayed by the electrocardiography acquisition apparatus 100 through an electronic device, a graphical position corresponding relationship between the first position and the second position of the predetermined electrocardiographic lead can be displayed on a display interface of the electronic device, wherein the electronic device includes but is not limited to a smart phone, a tablet computer, and a smart wearable device.

It can be understood that the position corresponding relationship of the predetermined electrocardiographic lead can be a displayed graphical position corresponding relationship, which is more intuitive for the user to obtain the position of the target object at which position the lead electrode of the predetermined electrocardiographic lead is connected, while acquiring the first electrocardiographic data, and the position of the target object to be connected at while acquiring the second electrocardiographic data.

It can also be understood that the first position icon and the second position icon are configured to simulate the lead electrode of the predetermined electrocardiographic lead. Specifically, the first position icon is configured to simulate the lead electrode of the predetermined electrocardiographic lead while acquiring the first electrocardiographic data, and the second position icon is configured to simulate the lead electrode of the predetermined electrocardiographic lead while acquiring the second electrocardiographic data.

As shown in FIG. 4, if the predetermined electrocardiographic lead, whose electrode position is to be adjusted, is determined to include lead V1, lead V2, and lead V3, the first position icon, which corresponds to an initial electrode position on the target object which position corresponds to lead V1, lead V2, and lead V3 respectively, is displayed on the first image element. For example, the first position icon includes an icon V1 which corresponds to lead V1, an icon V2 which corresponds to lead V2, and an icon V3 which corresponds to lead V3.

At the same time, the second position icon and a connection guidance for a movement between the first position and the second position are displayed on the first image element, wherein the second position icon corresponds to the second positions on the target object, at which positions the electrodes which correspond to lead V1, lead V2 and lead V3 are to be connected. Wherein, the second position icon includes an icon 3R which corresponds to the lead V3R, an icon 4R which corresponds to the lead V4R, and an icon 5R which corresponds to the lead V5R.

Specifically, the first image element includes a limb image element which is configured to indicate human limbs, and a chest image element which is configured to indicate human chest, wherein a position icon of the electrode which corresponds to the limb lead is set at a limb image element, so as to indicate that, during acquiring electrocardiographic data, the RA electrode which corresponds to the limb lead is connected at the right upper limb of the target object, the LA electrode which corresponds to the limb lead is connected at the left upper limb of the target object, the LL electrode which corresponds to the limb lead is connected at the left lower limb of the target object, and the RL electrode which corresponds to the limb lead is connected at the right lower limb of the target object.

The position icons of the electrodes which correspond to lead V1, lead V2, and lead V3 are set at a chest image element, so as to indicate that while acquiring first electrocardiographic data, the electrode which corresponds to lead V1 is connected at the position V1 of the target object, the electrode which corresponds to lead V2 is connected at the position V2 of the target object, and the electrode which corresponds to lead V3 is connected at the position V3 of the target object.

While acquiring second electrocardiographic data, the electrode which corresponds to lead V1 should be connected at the position 3R of the target object, the electrode which corresponds to lead V2 should be connected at the position 4R of the target object, and the electrode which corresponds to lead V3 should be connected at the position 5R of the target object.

At the same time, a movement arrow is arranged between the position V1 and the position 3R to guide the user to move the electrode which corresponds to lead V1 to the position 3R which corresponds to the lead V3R, so as to use said electrode as the electrode of the lead V3R.

A movement arrow is arranged between the position V2 and the position 4R to guide the user to move the electrode which corresponds to lead V2 to the position 4R which corresponds to the lead V4R, so as to use said electrode as the electrode of the lead V4R.

A movement arrow is arranged between the position V3 and the position 5R to guide the user to move the electrode which corresponds to lead V3 to the position 5R which corresponds to lead V5R, so as to use said electrode as the electrode of the lead V5R, thus achieving a precise position adjustment of the predetermined electrocardiographic lead.

As shown in FIG. 5, if the predetermined electrocardiographic lead, whose electrode position is to be adjusted, is determined to include lead V4, lead V5, and lead V6, the first position icon, which corresponds to an initial electrode position on the target object which position corresponds to lead V4, lead V5, and lead V6 respectively, is displayed on the first image element. For example, the first position icon includes an icon V4 which corresponds to the lead V4, an icon V5 which corresponds to the lead V5, and an icon V6 which corresponds to the lead V6.

At the same time, the second position icon and a connection guidance for a movement between the first position and the second position are displayed on the first image element, wherein the second position icon corresponds to the second positions on the target object, at which positions the electrodes which correspond to lead V4, lead V5 and lead V6 are to be connected. Wherein, the second position icon includes an icon V7 which corresponds to the lead V7, an icon V8 which corresponds to the lead V8, and an icon V9 which corresponds to the lead V9.

Specifically, the first image element includes a limb image element which is configured to indicate human limbs, a chest image element which is configured to indicate human chest, and a back image element which is configured to indicate human back.

Wherein, a position icon of the electrode which corresponds to the limb lead is set at the limb image element, so as to indicate that, during acquiring electrocardiographic data, the RA electrode which corresponds to the limb lead is connected at the right upper limb of the target object, the LA electrode which corresponds to the limb lead is connected at the left upper limb of the target object, the LL electrode which corresponds to the limb lead is connected at the left lower limb of the target object, and the RL electrode which corresponds to the limb lead is connected at the right lower limb of the target object.

The position icons of the electrodes which correspond to lead V4, lead V5, and lead V6 are set at the chest image element, so as to indicate that, during acquiring first electrocardiographic data, the electrode which corresponds to lead V4 is connected at the position V4 of the target object, the electrode which corresponds to lead V5 is connected at the position V5 of the target object, and the electrode which corresponds to lead V6 is connected at the position V6 of the target object.

While acquiring second electrocardiographic data, the electrode which corresponds to lead V4 should be connected at the position V9 of the target object, the electrode which corresponds to lead V5 should be connected at the position V8 of the target object, and the electrode which corresponds to lead V6 should be connected at the position V7 of the target object.

At the same time, a movement arrow is arranged between the position V4 and the position V9 to guide the user to move the electrode which corresponds to lead V4 to the position V9 which corresponds to lead V9, so as to use said electrode as the electrode of the lead V9.

A movement arrow is arranged between the position V5 and the position V8 to guide the user to move the electrode which corresponds to lead V5 to the position V8 which corresponds to lead V8, so as to use said electrode as the electrode of the lead V8.

At the same time, a movement arrow is arranged between the position V6 and the position V7 to guide the user to move the electrode which corresponds to lead V6 to the position V7 which corresponds to lead V7, so as to use said electrode as the electrode of the lead V7, thus achieving a precise position adjustment of the predetermined electrocardiographic lead.

As shown in FIG. 6, similarly, if the predetermined electrocardiographic lead, whose electrode position is to be adjusted, is determined to include leads V1 to V6, the first position icon, which corresponds to an initial electrode position on the target object which position corresponds to lead V1, lead V2, lead V3, lead V4, lead V5, and lead V6, is displayed on the first image element. For example, the first position icon includes an icon V1 which corresponds to the lead V1, an icon V2 which corresponds to the lead V2, an icon V3 which corresponds to the lead V3, an icon V4 which corresponds to the lead V4, an icon V5 which corresponds to the lead V5, and an icon V6 which corresponds to the lead V6.

At the same time, the leads V1 to V6, as well as corresponding second position icons are displayed on the first image element, wherein the second position icons correspond to the second positions on the target object, at which positions the electrodes, which correspond to the leads V1 to V6, are to be connected. Wherein, the second position icons include an icon 3R which corresponds to the lead V3R, an icon 4R which corresponds to the lead V4R, an icon 5R which corresponds to the lead V5R, an icon V7 which corresponds to the lead V7, an icon V8 which corresponds to the lead V8, and an icon V9 which corresponds to the lead V9.

Specifically, the first image element includes a limb image element which is configured to indicate human limbs, a chest image element which is configured to indicate human chest, and a back image element which is configured to indicate human back.

Wherein, a position icon of the electrode which corresponds to the limb lead is set at the limb image element, so as to indicate that, during acquiring electrocardiographic data, the RA electrode which corresponds to the limb lead is connected at the right upper limb of the target object, the LA electrode which corresponds to the limb lead is connected at the left upper limb of the target object, the LL electrode which corresponds to the limb lead is connected at the left lower limb of the target object, and the RL electrode which corresponds to the limb lead is connected at the right lower limb of the target object.

The position icons of the electrodes which correspond to the leads V1~V6 are set at the chest image element, so as to indicate that during acquiring first electrocardiographic data, the electrode which corresponds to lead V1 is connected at the position V1 of the target object, the electrode which corresponds to lead V2 is connected at the position V2 of the target object, the electrode which corresponds to lead V3 is connected at the position V3 of the target object, the electrode which corresponds to lead V4 is connected at the position V4 of the target object, the electrode which corresponds to lead V5 is connected at the position V5 of the target object, and the electrode which corresponds to lead V6 is connected at the position V6 of the target object.

While acquiring second electrocardiographic data, the electrode which corresponds to lead V1 should be connected at the position 3R of the target object, the electrode which corresponds to lead V2 should be connected at the position 4R of the target object, the electrode which corresponds to lead V3 should be connected at the position 5R of the target object, the electrode which corresponds to lead V4 should be connected at the position V9 of the target object, the electrode which corresponds to lead V5 should be connected at the position V8 of the target object, and the electrode which corresponds to lead V6 should be connected at the position V7 of the target object.

At the same time, a movement arrow is arranged between the position V1 and the position 3R to guide the user to move the electrode which corresponds to lead V1 to the position 3R which corresponds to the lead V3R, so as to use said electrode as the electrode of the lead V3R.

A movement arrow is arranged between the position V2 and the position 4R to guide the user to move the electrode which corresponds to lead V2 to the position 4R which corresponds to the lead V4R, so as to use said electrode as the electrode of the lead V4R.

A movement arrow is arranged between the position V3 and the position 5R to guide the user to move the electrode which corresponds to lead V3 to the position 5R which corresponds to lead V5R, so as to use said electrode as the electrode of the lead V5R, thus achieving a precise position adjustment of the predetermined electrocardiographic lead.

A movement arrow is arranged between the position V4 and the position V9 to guide the user to move the electrode which corresponds to lead V4 to the position V9 which corresponds to lead V9, so as to use said electrode as the electrode of the lead V9.

A movement arrow is arranged between the position V5 and the position V8 to guide the user to move the electrode which corresponds to lead V5 to the position V8 which corresponds to lead V8, so as to use said electrode as the electrode of the lead V8.

A movement arrow is arranged between the position V6 and the position V7 to guide the user to move the electrode which corresponds to lead V6 to the position V7 which corresponds to lead V7, so as to use said electrode as the electrode of the lead V7, thus achieving a precise position adjustment of the predetermined electrocardiographic lead.

Alternatively, the movement guidance information at least includes one of: movement guidance text, movement guidance path, and movement guidance direction.

Alternatively, icon attributes of at least some icons in the first position icon and/or in the second position icon are different, wherein the icon attribute comprises at least one of: an icon color, an icon shape, and an icon size.

For example, the icon attributes of at least some icons in the first position icon are different, or the icon attributes of at least some icons in the second position icon are different, or the icon attributes of the first position icon and of the second position icon are different; wherein the icon attribute includes at least one of: an icon color, an icon shape, and an icon size. Each position icon is identified by different icon attributes, which is convenient for quickly distinguishing the position icons.

Alternatively, the position corresponding relationship of the predetermined electrocardiographic lead also includes a first position name which corresponds to the first position, a second position name which corresponds to the second position, wherein the first position name is different from the second position name.

For example, if the additional leads operation for right chest is needed, that is, the predetermined electrocardiographic lead that needs to be moved includes lead V1, lead V2, and lead V3, then the first position name can be V1 corresponding to lead V1, V2 corresponding to lead V2, and lead V3 corresponding to lead V3, and the second position name can be 3R corresponding to lead V3R, 4R corresponding to lead V4R, and 5R corresponding to lead V5R.

Similarly, if the additional leads operation for left posterior chest wall is needed, that is, the predetermined electrocardiographic lead that needs to be moved includes lead V4, lead V5, and lead V6, then the first position name can be V4 corresponding to lead V4, V5 corresponding to lead V5, and V6 corresponding to lead V6, and the second position name can be V9 corresponding to lead V9, V8 corresponding to lead V8, and V7 corresponding to lead V7.

It can be understood that the naming of the position name can be adjusted according to requirements, just for the user to know name information of the electrode of the predetermined electrocardiographic lead before and after adjustment.

In some embodiments, a display method of the position corresponding relationship of the predetermined electrocardiographic lead at least includes one of: at least displaying through a static image, at least displaying through a dynamic image, and at least displaying through a video.

Alternatively, the mode for displaying at least through a static image includes displaying by combining the static image with at least one of text and voice. For example, the first image element, the first position icon, the second position icon, and the movement guidance information are displayed through a static image. Alternatively, the first image element, the first position icon, and the second position icon are displayed through a static image, and the movement guidance information is displayed through text. Alternatively, the first image element, the first position icon and the second position icon are displayed through a static image, and the movement guidance information is outputted through voice broadcast.

Alternatively, the mode for displaying at least through a dynamic image includes displaying by combining the dynamic image with at least one of text and voice. For example, the first image element, the first position icon, the second position icon, and the movement guidance information are displayed through a dynamic image. Alternatively, the first image element, the first position icon and the second position icon are displayed through a dynamic image, and the movement guidance information is displayed through text. Alternatively, the first image element, the first position icon and the second position icon are displayed through a dynamic image, and the movement guidance information is outputted through voice broadcast.

Alternatively, the mode for displaying at least through a video includes displaying by combining a video with at least one of text and voice.

In some embodiments, the information entry at least includes a two-dimensional code, which is configured to display the position corresponding relationship between the first position and the second position on the display interface of the electronic device after being scanned by the electronic device.

It can be understood that the information entry can also be in other forms of coding. After the user scans the information entry through the electronic device, a graphical position corresponding relationship between the first position and the second position can be displayed on a display interface displayed on a display screen of the electronic device. Wherein, the electronic device includes but is not limited to a smart phone, a tablet computer, and a smart wearable device.

In some embodiments, after step S103, the method further includes outputting a conventional ECG according to the first electrocardiographic data or the historical electrocardiographic data, and/or outputting an additional ECG according to the second electrocardiographic data.

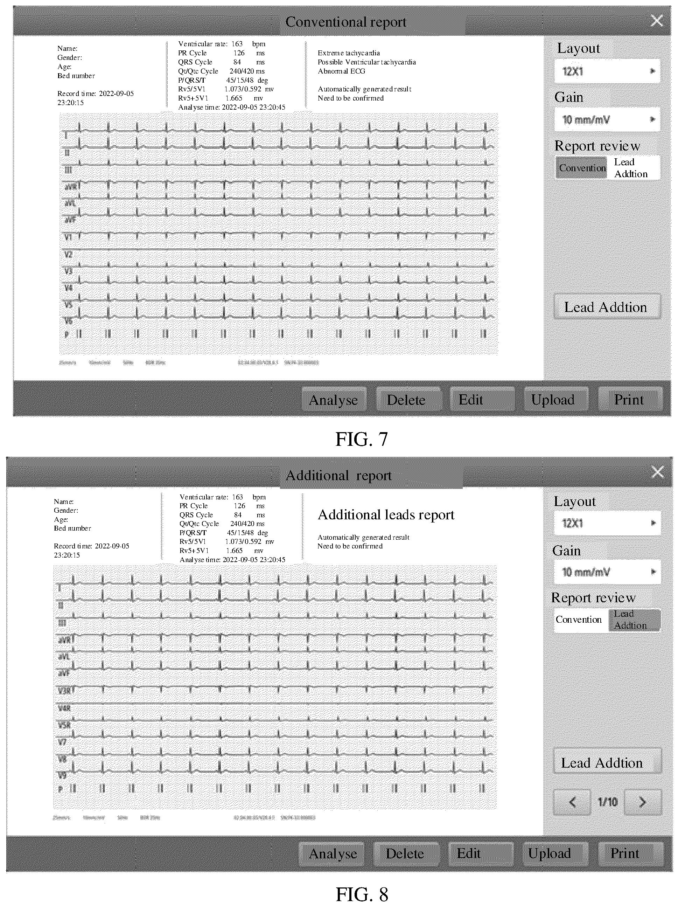

Refer to FIG. 7 and FIG. 8. For example, after acquiring the first electrocardiographic data or the historical electrocardiographic data, a conventional ECG is outputted according to the first electrocardiographic data or the historical electrocardiographic data. As shown in FIG. 7, the conventional ECG (also known as a conventional electrocardiographic report) includes electrocardiographic data (such as electrocardiographic waveform) corresponding to lead I, lead II, lead III, lead aVR, lead aVL, lead aVF and leads V1-V6.

After acquiring the second electrocardiographic data, additional ECG according to the second electrocardiographic data is outputted, as shown in FIG. 8. Additional ECG (also known as additional electrocardiographic report) is additional leads operation for right anterior chest and left posterior chest wall, then the electrocardiographic data (such as electrocardiographic waveform) corresponding to lead I, lead II, lead III, lead aVR, lead aVL, lead aVF, lead V3R, lead V4R, lead V5R, lead V7, lead V8, lead V9 are displayed.

In some embodiments, the conventional ECG includes a first name which corresponds to a predetermined electrocardiographic lead, and the additional ECG includes a second name which corresponds to the predetermined electrocardiographic lead, wherein the first name and the second name are different.

For example, additional ECG data is acquired through reusing the lead electrode which corresponds to the predetermined electrocardiographic lead. In the conventional ECG, the name of the predetermined electrocardiographic lead is the first name. In the additional ECG, the electrocardiography acquisition apparatus can determine the second name of the predetermined electrocardiographic lead in the additional ECG according to the received addition instruction, and modify the first name of the predetermined electrocardiographic lead to the second name when generating the additional ECG.

For example, taking the additional leads operation for right chest as an example, the predetermined electrocardiographic leads that need to be reused are lead V1, lead V2 and lead V3, and names of the predetermined electrocardiographic leads in the conventional ECG are lead V1, lead V2 and lead V3. In the additional ECG, the electrode which corresponds to lead V1 is reused as the electrode of lead V3R, the electrode which corresponds to lead V2 is reused as the electrode of lead V4R, and the electrode which corresponds to lead V3 is reused as the electrode of lead V5R. Then, when generating the additional ECG, lead V1 is modified into lead V3R, lead V2 is modified into lead V4R, and lead V3 is modified into lead V5R, so that the medical staff can know that the electrocardiographic data obtained by the current additional ECG is the electrocardiographic data corresponding to the additional leads operation for right chest.

In some embodiments, after outputting a conventional ECG according to the first electrocardiographic data or the historical electrocardiographic data and outputting an additional ECG according to the second electrocardiographic data, the method further includes:
displaying the conventional ECG, and when a first switch instruction is received, switching to display the additional ECG from the conventional ECG; or displaying the additional ECG, and when a second switch instruction is received, switching to display the conventional ECG from the additional ECG to the conventional ECG.

For example, the first switch instruction and the second switch instruction are instructions generated by the user through triggering the interaction unit 40. The interaction unit 40 may be a physical key set on the electrocardiography acquisition host 10, or a remote controller in communicative connection with the electrocardiography acquisition host 10, or a predetermined area on the display unit 101, or a virtual key set on the display interface which corresponds to the display unit 101.

For example, a preview interface of electrocardiographic report is set with a virtual key for switching between displaying the conventional ECG and displaying the additional ECG. The user can trigger the virtual key by clicking, double clicking, pressing for a preset duration, sliding, etc., so as to realize the switching between displaying the conventional ECG and displaying the additional ECG.

As shown in FIG. 7, trigger a virtual button of "Convention" under an option of "view report" on the preview interface of electrocardiographic report, so as to display the conventional electrocardiographic report of the target object on the interface.

As shown in FIG. 8, trigger a virtual button of "Addition" under an option of "view report" on the preview interface of electrocardiographic report, so as to display the additional electrocardiographic report of the target object on the interface.

In some embodiments, the first acquisition instruction is generated by an external object (e.g., user) through triggering the first electrocardiography acquisition entry of the interaction unit 40, and the second acquisition instruction is generated by an external object (e.g., user) through triggering the second electrocardiography acquisition entry of the interaction unit 40. Wherein, the first electrocardiography acquisition entry includes at least one of: a button which is arranged at the electrocardiography acquisition apparatus 100, a touch-control element which is arranged at the electrocardiography acquisition apparatus 100, and a virtual icon which is displayed on the display interface of the electrocardiography acquisition apparatus 100; and /or the second electrocardiography acquisition entry includes at least one of: a button which is arranged at the electrocardiography acquisition apparatus 100, a touch-control element which is arranged at the electrocardiography acquisition apparatus 100, and a virtual icon which is displayed on the display interface of the electrocardiography acquisition apparatus 100.

In some embodiments, the first electrocardiography acquisition entry is a first virtual icon which is displayed on the display interface of the electrocardiography acquisition apparatus 100, and the second electrocardiography acquisition entry is a second virtual icon which is displayed on the display interface of the electrocardiography acquisition apparatus 100, wherein the first virtual icon and the second virtual icon can be simultaneously displayed on a same display interface of the electrocardiography acquisition apparatus 100.

In some embodiments, the display interface of the electrocardiography acquisition apparatus 100 includes a real-time electrocardiography interface for displaying real-time electrocardiographic data of a target object, a report preview interface for previewing an electrocardiographic report which is generated from the real-time electrocardiographic data, and a historical electrocardiography interface for displaying historical electrocardiographic data; wherein the first virtual icon and the second virtual icon are simultaneously displayed on at least one of the real-time electrocardiography interface, the report preview interface, and the historical electrocardiography interface.

In some embodiments, the display interface of the electrocardiography acquisition apparatus includes a real-time electrocardiography interface for displaying real-time electrocardiographic data which is acquired by the lead electrode(s) of the multiple electrocardiographic leads, a report preview interface for previewing an electrocardiographic report which is generated from the real-time electrocardiographic data, and a historical electrocardiography interface for displaying historical electrocardiographic data.

The addition instruction is generated by an external object (e.g., user) through triggering an addition entry; wherein the addition entry includes at least one of: a button which is arranged at the electrocardiography acquisition apparatus 100, a touch-control element which is arranged at the electrocardiography acquisition apparatus 100, and a virtual icon which is displayed on the display interface of the electrocardiography acquisition apparatus 100; wherein the addition entry is arranged on at least one of the real-time electrocardiography interface, the report preview interface, and the historical electrocardiography interface.

When the addition entry, which is arranged on the report preview interface or the historical electrocardiography interface, is triggered, a data association is established between the second electrocardiographic data and the first electrocardiographic data, so as to display, in a same electrocardiographic report, an ECG which corresponds to the first electrocardiographic data and an ECG which corresponds to the second electrocardiographic data.

When the addition entry, which is arranged on the real-time electrocardiography interface, is triggered, an ECG which corresponds to the first electrocardiographic data and an ECG which corresponds to the second electrocardiographic data are displayed in different electrocardiographic reports during outputting the electrocardiographic reports.

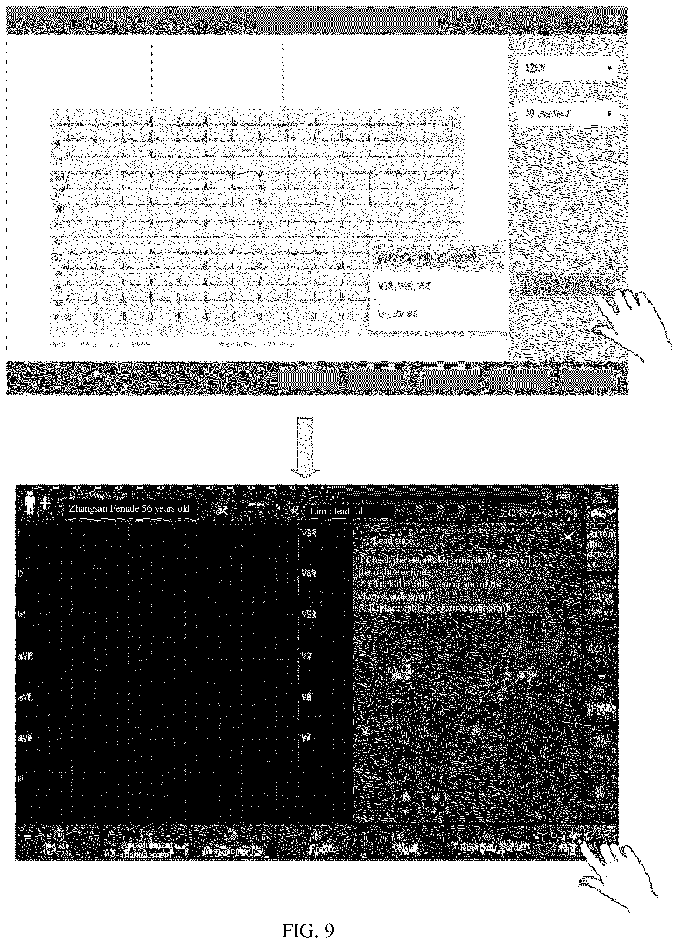

Referring to FIG. 9, for example, during previewing a conventional electrocardiographic report on the report preview interface of the electrocardiography acquisition apparatus 100, if additional electrocardiographic data is required to be acquired, the addition entry (such as an virtual button of "Addition"), which is arranged on the report preview interface, can be triggered to select the additional electrocardiographic data which is required to be acquired, and to generate a corresponding addition instruction. Such that, the electrocardiography acquisition apparatus 100 responds to the addition instruction and displays the adjustment guidance for electrode position, which guidance is adapted to said addition instruction.

The adjustment guidance for electrode position is used to display a position corresponding relationship between the first position and the second position of the predetermined electrocardiographic lead in multiple electrocardiographic leads, so as to enable a user to adjust a position of each lead electrode, which is connected with the target object, according to the adjustment guidance for electrode position.

After the user completes the adjustment of each lead electrode according to the adjustment guidance for electrode position, the second acquisition instruction can be generated by triggering the second electrocardiography acquisition entry on the display interface (such as a virtual button of "start acquire"), so as to acquire second electrocardiographic data, that is, to acquire additional electrocardiographic data.

At this time, a data association is established between the second electrocardiographic data of the target object acquired by the electrocardiography acquisition apparatus 100 and the first electrocardiographic data that has been acquired. After the data association between the second electrocardiographic data and the first electrocardiographic data is established, during outputting an electrocardiographic report, an ECG which corresponds to the first electrocardiographic data and an ECG which corresponds to the second electrocardiographic data are displayed in a same electrocardiographic report, so that the medical staff can obtain relatively complete electrocardiographic data of the target object in the same report.

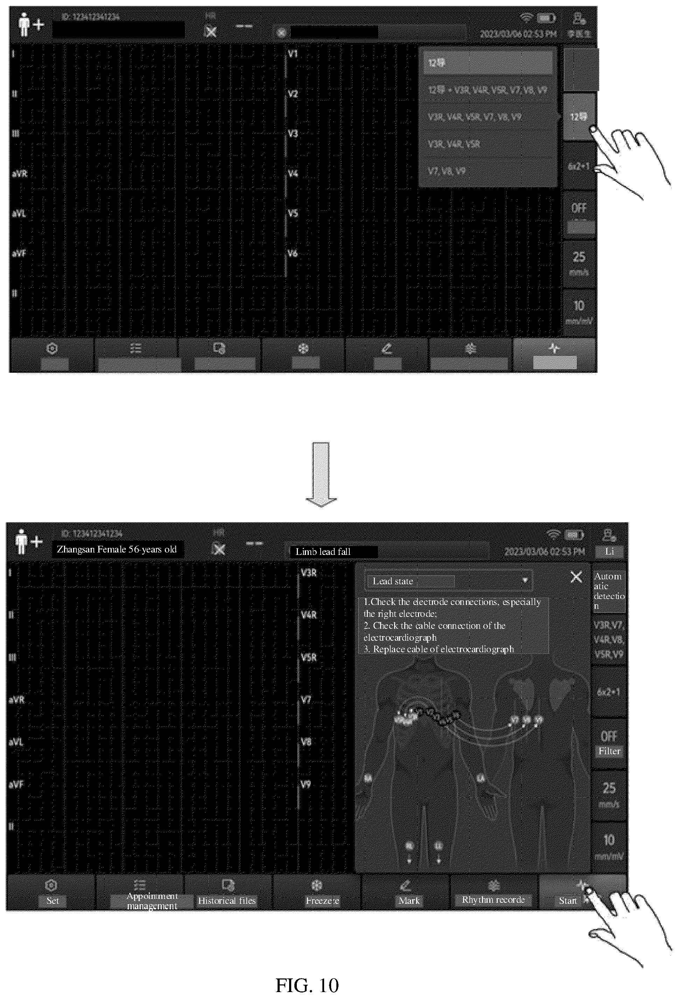

Refer to FIG. 10. When the addition entry, which is arranged on the real-time electrocardiography interface (such as options V3R, V4R, V5R, V7, V8, V9 under a button of "12-lead", or options V3R, V4R, V5R under a button of "12-lead", or options V7, V8, V9 options under a button of "12-lead") is triggered, a corresponding addition instruction is generated, so as to enable the electrocardiography acquisition apparatus 100 to respond to the addition instruction and display the adjustment guidance for electrode position, which guidance is adapted to the addition instruction.

The adjustment guidance for electrode position is used to display a position corresponding relationship between the first position and the second position of the predetermined electrocardiographic lead in multiple electrocardiographic leads, so as to enable a user to adjust a position of each lead electrode, which is connected with the target object, according to the adjustment guidance for electrode position.

After the user completes the adjustment of each lead electrode according to the adjustment guidance for electrode position, the second acquisition instruction can be generated by triggering the second electrocardiography acquisition entry on the display interface (such as a virtual button of "start acquire") to acquire second electrocardiographic data.

After acquisition of the second electrocardiographic data is completed, an ECG which corresponds to the first electrocardiographic data and an ECG which corresponds to the second electrocardiographic data are displayed in different electrocardiographic reports during outputting the electrocardiographic reports.

Refer to FIG. 10 again. When the addition entry (such as 12-lead option under a button of "12-lead") which is arranged on the real-time electrocardiography interface, is triggered, a position guidance for conventional electrode, which guidance corresponds to the conventional leads (12-lead), is displayed on the display interface of the electrocardiography acquisition apparatus 100; wherein the position guidance for conventional electrode includes a position of the target object at which position the lead electrode which corresponds to the conventional 12-lead is connected.

As shown in FIG. 3, the position guidance for conventional electrode displays, on a corresponding display interface, positions at the target object, at which positions four electrodes are connected, which electrodes correspond to the limb leads. For example, the RA electrode of a non-predetermined electrocardiographic lead is connected at the right upper limb of the target object, the LA electrode of a non-predetermined electrocardiographic lead is connected at the left upper limb of the target object, the LL electrode of a non-predetermined electrocardiographic lead is connected at the left lower limb of the target object, and the RL electrode of a non-predetermined electrocardiographic lead is connected at the right lower limb of the target object. The limb lead data of the target object is obtained through the four lead electrodes.

At the same time, leads V1-V6 are displayed, which are connected at six corresponding preset positions at the front chest of the target object. An electrode which corresponds to lead V1 in multiple lead electrodes 20 is connected at a position V1 of a fourth intercostal space on a right side of human sternum, an electrode which corresponds to lead V2 is connected at a position V2 of a fourth intercostal space on a left side of human sternum, an electrode which corresponds to lead V4 is connected at a position V4 of a midclavicular line of a fifth intercostal space on the left side of human sternum, and an electrode which corresponds to lead V3 is connected at a position V3 between leads V2 and V4, an electrode which corresponds to lead V5 is connected at a position V5 at a point of intersection between a fifth intercostal space of human sternum and an anterior axillary line, and an electrode which corresponds to lead V6 is connected at a point of intersection between a fifth intercostal space of human sternum and a midaxillary line.

After the lead electrode is connected, the user transmits a first acquisition instruction to the electrocardiography acquisition apparatus 100 by triggering the interaction unit 40 or the first electrocardiography acquisition entry, so as to enable the electrocardiography acquisition apparatus 100 to acquire, in response to the electrocardiography acquisition instruction, first electrocardiographic data of the target object.

Further refer to FIG. 10. When the addition entry, which is arranged on the real-time electrocardiography interface (such as options of 12-lead and of V3R, V4R, V5R, V7, V8, V9 under a button of "12-lead") is triggered, the electrocardiography acquisition apparatus 100 determines that the user needs to acquire 18-lead electrocardiographic data of the target object by default, and displays, on a display interface, position guidance for conventional electrode which guidance corresponds to the conventional leads (12-lead), so as to enable the user to complete acquisition of the conventional electrocardiographic data of the target object (also known as the first electrocardiographic data).

After acquisition of the first electrocardiographic data is completed, an addition entry is displayed on a corresponding display interface. For example, a virtual icon of "next" or "addition" is displayed on the corresponding display interface. When the user triggers the addition entry, the electrocardiography acquisition apparatus 100 receives a third addition instruction (for example, an addition instruction for right chest lead and left posterior chest wall lead). According to the received third addition instruction, the electrocardiography acquisition apparatus 100 displays a third adjustment guidance for electrode position. The third adjustment guidance for electrode position shows that leads, whose electrode position needs to be adjusted for the additional leads operation on the right chest and the left posterior chest wall, include lead V1, lead V2, lead V3, lead V4, lead V5, and lead V6.

**In** such a way, the user is assisted to efficiently and accurately complete a connection between the lead electrode and the target object, which enables the user to efficiently obtain the second electrocardiographic data of the target object. Based on the acquisition of the first electrocardiographic data and the second electrocardiographic data of the target object, the 18-lead electrocardiographic data of the target object is obtained.

**In** some embodiments, the method further includes: also displaying, by the electrocardiography acquisition apparatus, in response to the addition instruction, a third position icon which corresponds to a third position, at which position of the target object a non-predetermined electrocardiographic lead (also known as limb lead) in multiple electrocardiographic leads is connected, and the third position icon is configured to indicate a reference connection position between the lead electrode of the non-predetermined electrocardiographic lead and the target object.

When the lead electrode of the predetermined electrocardiographic lead is connected at a position of the target object, which position is indicated by the first position, and the lead electrode of the non-predetermined electrocardiographic lead is connected at the reference connection position of the target object, the electrocardiography acquisition apparatus can acquire first electrocardiographic data of the target object through lead electrodes of multiple electrocardiographic leads.

Wherein, the non-predetermined electrocardiographic lead is a lead, whose electrode position does not need to be adjusted, among the multiple electrocardiographic leads, which are connected with the target object. Therefore, the non-predetermined electrocardiographic lead is a fixed electrocardiographic lead which is connected at a fixed position of the human body of the target object, that is, the non-predetermined electrocardiographic lead is the limb lead.

For example, after receiving an addition instruction, the electrocardiography acquisition apparatus 100, in response to the addition instruction, displays at least one of: a position corresponding relationship between a first position and a second position of a predetermined electrocardiographic lead in multiple electrocardiographic leads, an information entry which is capable of viewing a position corresponding relationship between a first position and a second position, and a third position of an non-predetermined electrocardiographic lead in the multiple electrocardiographic leads. Wherein, the first position is configured to indicate an initial connection position between a lead electrode of a predetermined electrocardiographic lead of the electrocardiography acquisition apparatus and the target object, the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; the third position is configured to indicate a reference connection position between the lead electrode of the non-predetermined electrocardiographic lead and the target object.

When the lead electrode of the predetermined electrocardiographic lead is connected at the initial connection position of the target object, and the lead electrode of the non-predetermined electrocardiographic lead is connected at the reference connection position of the target object, the electrocardiography acquisition apparatus can acquire first electrocardiographic data of the target object through lead electrodes of multiple electrocardiographic leads; wherein the initial connection position is a position of the target object at which position the lead electrode of the predetermined electrocardiographic lead needs to be connected while acquiring conventional electrocardiography.

As shown in FIGS. 4 to 6, after receiving the addition instruction, the electrocardiography acquisition apparatus 100 displays not only a position corresponding relationship between a first position and a second position of the predetermined electrocardiographic lead to be adjusted, but also a third position relationship of the non-predetermined electrocardiographic lead that does not need to be adjusted. That is, display positions at the target object, at which positions four electrodes are connected, which electrodes correspond to the limb leads. For example, the RA electrode of a non-predetermined electrocardiographic lead is connected at the right upper limb of the target object, the LA electrode of a non-predetermined electrocardiographic lead is connected at the left upper limb of the target object, the LL electrode of a non-predetermined electrocardiographic lead is connected at the left lower limb of the target object, and the RL electrode of a non-predetermined electrocardiographic lead is connected at the right lower limb of the target object. The limb lead data of the target object is obtained through the four lead electrodes.

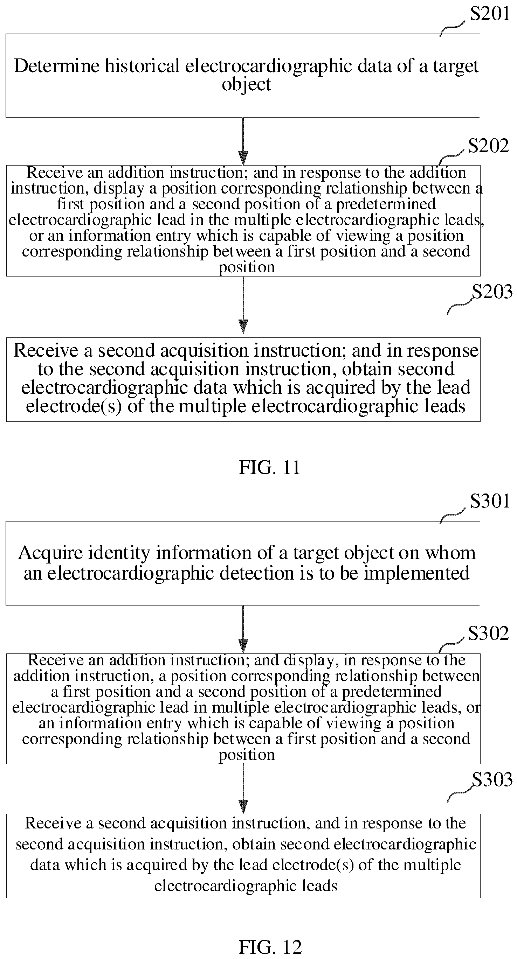

Please refer to FIG. 11, which is a flow chart for steps of an electrocardiography acquisition method provided by a second embodiment of the disclosure.

As shown in FIG. 11, the electrocardiography acquisition method includes steps S201 to S203.

Step S201: determine historical electrocardiographic data of a target object.

Step S202: receive an addition instruction; and display, in response to the addition instruction, a position corresponding relationship between a first position and a second position of a predetermined electrocardiographic lead in multiple electrocardiographic leads, or an information entry which is capable of viewing a position corresponding relationship between a first position and a second position; wherein the historical electrocardiographic data is data acquired by lead electrode(s) of the multiple electrocardiographic leads and obtained by the electrocardiography acquisition apparatus in response to a first acquisition instruction; wherein the first position is configured to indicate a position of the target object at which position a lead electrode of the predetermined electrocardiographic lead is connected while acquiring the historical electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position.

Step S203: receive a second acquisition instruction, and in response to the second acquisition instruction, obtain second electrocardiographic data which is acquired by the lead electrode(s) of the multiple electrocardiographic leads. For example, different from the first embodiment, the target object for acquiring additional electrocardiographic data is determined by historical electrocardiographic data in this embodiment.

The historical data of the target object at least includes identity information and historical electrocardiographic data, wherein, the historical electrocardiographic data at least includes conventional electrocardiographic data of the target object, which is acquired in history, also known as the first electrocardiographic data of the target object.

When the historical data of the target object has been stored in a database (e.g., an electrocardiographic database), if more electrocardiographic data of the target object is required, the historical data of the target object can be determined from the database, so as to obtain the identity information of the target object, and then the user does not need to input the identity information of the target object again during acquiring additional electrocardiographic data.

After determining the target object for acquiring additional electrocardiographic data, the user triggers the addition entry (such as an virtual button of "addition" ) which is arranged on a corresponding display interface of the electrocardiography acquisition apparatus 100, selects additional electrocardiographic data to be acquired, and generates a corresponding addition instruction, so as to enable the electrocardiography acquisition apparatus 100 to display, in response to the addition instruction, an adjustment guidance for electrode position, which guidance is adapted to the addition instruction.

The adjustment guidance for electrode position is used to display a position corresponding relationship between the first position and the second position of the predetermined electrocardiographic lead in multiple electrocardiographic leads, so as to enable a user to adjust a position of each lead electrode, which is connected with the target object, according to the adjustment guidance for electrode position.

After the user completes the adjustment of each lead electrode according to the adjustment guidance for electrode position, the second acquisition instruction can be generated by triggering the second electrocardiography acquisition entry on the display interface (such as a virtual button of "start acquire") to acquire second electrocardiographic data.

Please refer to FIG. 12, which is a flow chart for steps of an electrocardiography acquisition method provided by a third embodiment of the disclosure.

As shown in FIG. 12, the electrocardiography acquisition method includes steps S301 to S303.

Step S301: acquire identity information of a target object on whom an electrocardiographic detection is to be implemented.

Step S302: receive an addition instruction; and display, in response to the addition instruction, a position corresponding relationship between a first position and a second position of a predetermined electrocardiographic lead in multiple electrocardiographic leads, or an information entry which is capable of viewing a position corresponding relationship between a first position and a second position; wherein the first position is configured to indicate an initial connection position between a lead electrode of the predetermined electrocardiographic lead of the electrocardiography acquisition apparatus and the target object, the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; wherein the initial connection position is a position of the target object at which position the lead electrode of the predetermined electrocardiographic lead needs to be connected while acquiring conventional electrocardiography.

Step S303: receive a second acquisition instruction, and in response to the second acquisition instruction, obtain second electrocardiographic data which is acquired by the lead electrode(s) of the multiple electrocardiographic leads.

For example, the method for acquiring the identity information of the target object can be as follows: inputting the identity information of the target object by a user through an input device, wherein the input device includes but is not limited to at least one of: a keyboard, a mouse, a touch panel and a button; or obtaining, from a corresponding database, the identity information of the target object, by a user though manipulating the electrocardiography acquisition apparatus. For example, the electrocardiographic database can be a cloud database or a local database, and there is no restriction.

Different from the first embodiment, this embodiment directly acquires the additional electrocardiographic data of the target object by acquiring the identity information of the target object without acquiring the conventional electrocardiographic data of the target object first.

Additional electrocardiographic data includes data for a lead, such as lead V3R, lead V4R, and lead V5R, or data for a lead, such as lead V7, lead V8, and lead V9; or lead data for at least one lead, such as lead V3R, lead V4R, lead V5R, lead V7, lead V8, and lead V9.

Refer to FIG. 10. When the addition entry, which is arranged on the real-time electrocardiography interface (such as options V3R, V4R, V5R, V7, V8, V9 under a button of "12-lead", or options V3R, V4R, V5R under a button of "12-lead", or options V7, V8, V9 options under a button of "12-lead") is triggered, a corresponding addition instruction is generated, so as to enable the electrocardiography acquisition apparatus 100 to respond to the addition instruction and display the adjustment guidance for electrode position, which guidance is adapted to the addition instruction.

The adjustment guidance for electrode position is used to display a position corresponding relationship between the first position and the second position of the predetermined electrocardiographic lead in multiple electrocardiographic leads, so as to enable a user to adjust a position of each lead electrode, which is connected with the target object, according to the adjustment guidance for electrode position.

For example, when the options of V3R, V4R and V5R under the "12-lead" button are triggered, an addition instruction is generated to acquire lead data of lead V3R, lead V4R and lead V5R, and an adjustment guidance for electrode position as shown in FIG. 4 is generated to guide the user to adjust each lead electrode.

Alternatively, when the options of V7, V8 and V9 under the "12-lead" button are triggered, an addition instruction is generated to acquire lead data of lead V7, lead V8 and lead V9, and an adjustment guidance for electrode position as shown in FIG. 5 is generated to guide the user to adjust each lead electrode.

Alternatively, when the options of V3R, V4R, V5R, V7, V8, V9 under the "12-lead" button are triggered, an addition instruction is generated to acquire lead data of lead V3R, lead V4R, lead V5R, lead V7, lead V8, lead V9, etc., and an adjustment guidance for electrode position as shown in FIG. 6 is generated to guide the user to adjust each lead electrode.

After the user completes the adjustment of each lead electrode according to the adjustment guidance for electrode position, the second acquisition instruction can be generated by triggering the second electrocardiography acquisition entry on the display interface (such as a virtual button of "start acquire") to acquire second electrocardiographic data.

After acquisition of the second electrocardiographic data is completed, an ECG which corresponds to the first electrocardiographic data and an ECG which corresponds to the second electrocardiographic data are displayed in different electrocardiographic reports of the target object, during outputting the electrocardiographic reports.

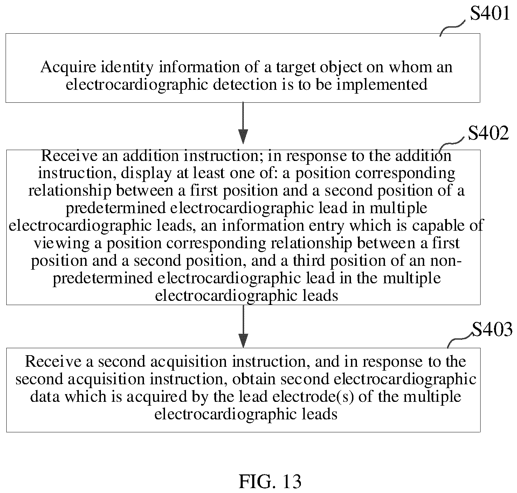

Please refer to FIG. 13, which is a flow chart for steps of an electrocardiography acquisition method provided by a fourth embodiment of the disclosure.

As shown in FIG. 13, the electrocardiography acquisition method includes steps S401 to S403.

Step S401: acquire identity information of a target object on whom an electrocardiographic detection is to be implemented.

Step S402: receive an addition instruction; in response to the addition instruction, display at least one of: a position corresponding relationship between a first position and a second position of a predetermined electrocardiographic lead in multiple electrocardiographic leads, an information entry which is capable of viewing a position corresponding relationship between a first position and a second position, and a third position of an non-predetermined electrocardiographic lead in the multiple electrocardiographic leads. Wherein, the first position is configured to indicate an initial connection position between a lead electrode of a predetermined electrocardiographic lead of the electrocardiography acquisition apparatus and the target object, the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; the third position is configured to indicate a reference connection position between the lead electrode of the non-predetermined electrocardiographic lead and the target object.

Step S403: receive a second acquisition instruction, and in response to the second acquisition instruction, obtain second electrocardiographic data which is acquired by the lead electrode(s) of the multiple electrocardiographic leads.

For example, different from the third embodiment, the data displayed by the central electrical acquisition apparatus 100 in response to the addition instruction in this embodiment, also includes the third position of the non-predetermined electrocardiographic lead in the multiple electrocardiographic leads. The non-predetermined electrocardiographic lead is the limb lead, that is, lead I, lead II, lead III, lead aVR, lead aVL, lead aVF. The limb lead requires corresponding four lead electrodes 20 to be connected at preset portions of the limbs of the target object.

As shown in FIG. 4, the RA electrode in the multiple lead electrodes 20 is connected at the right upper limb of the target object, the LA electrode in the multiple lead electrodes 20 is connected at the left upper limb of the target object, the LL electrode in the multiple lead electrodes 20 is connected at the left lower limb of the target object, the LA electrode in the multiple lead electrodes 20 is connected at the left upper limb of the target object, and the limb lead data of the target object is obtained through the four lead electrodes.

During directly acquiring the additional electrocardiographic data of the target object, displaying the specific connection position between each lead and the target object can enable the user to efficiently and accurately connect the lead electrode which corresponds to each lead with the target object, so as to efficiently and accurately acquire the additional electrocardiographic data of the target object.

An embodiment of this disclosure also provides a storage medium for computer-readable storage. The storage medium stores one or more programs, which is capable of being executed by one or more processors, so as to realize steps of any electrocardiography acquisition method provided by the embodiment of the description of the disclosure.

The storage medium may be an internal storage unit of the electrocardiography acquisition apparatus of the preceding embodiment, such as a memory of the electrocardiography acquisition apparatus. The storage medium can also be an external storage device of the electrocardiography acquisition apparatus, such as a plug-in hard disk, a smart media card (SMC), a secure digital (SD) card, a flash card, etc., which is equipped on the electrocardiography acquisition apparatus.

Those skilled in the art can understand that all or some of the steps in the methods disclosed above and the functional modules/units in the device can be implemented as software, firmware, hardware and their appropriate combinations. In the hardware embodiment, the division between functional modules/units mentioned in the above description does not necessarily correspond to the division of physical components. For example, a physical component may have multiple functions, or a function or step may be performed by several physical components in cooperation. Some or all physical components may be implemented as software executed by a processor, such as a central processing unit, a digital signal processor, or a microprocessor, or by a hardware, such as an integrated circuit, an application specific integrated circuit. Such software may be distributed on a computer-readable medium (or a non-transient medium), which may include a computer storage medium, and a communication medium (or a transient medium). As known to those of ordinary skill in the art, the term "computer storage medium" includes volatile and non-volatile, removable and non-removable media, which is implemented in any method or technology for storing information, such as a computer-readable instruction, a data structure, a program module or other data. Computer storage media includes but is not limited to RAM, ROM, EEPROM, Flash or other memory technology, CD-ROM, Digital versatile disc (DVD) or other optical disk storage, a magnetic cartridge, a magnetic tape, a magnetic disk storage or other magnetic storage device, or any other medium that can be used to store desired information and can be accessed by a computer. In addition, it is well known to those of ordinary skill in the art that the communication medium generally contains a computer-readable instruction, a data structure, a program module, or other data in a modulated data signal, such as a carrier or other transmission mechanism, and may include any information delivery medium.

It should be understood that the terms used in the description of this disclosure are only for the purpose of describing specific embodiments and are not intended to limit the disclosure. As used in the description of this disclosure and the appended claims, unless the context clearly indicates other circumstances, the singular forms of "a", "an" and "the" are intended to include the plural form.

It should also be understood that the term "and/or" used in the description of this disclosure and the appended claims refers to any combination of one or more of the items listed in association and all possible combinations, and includes these combinations. It should be noted that in this article, the terms "include", "comprise" or any other variant thereof are intended to cover non-exclusive inclusion, so that a process, method, article or system that includes a series of elements includes not only those elements, but also other elements that are not explicitly listed, or elements inherent to such a process, method, article or system. In the absence of more restrictions, the element defined by the statement "including a..." does not exclude the existence of another identical element in the process, method, article or system that includes the element.

The above serial number of the embodiment of this disclosure is are used only for descriptive purposes and cannot be understood as indicating or implying relative importance or implicitly indicating the number of indicated technical features. The above is only the specific implementation of this disclosure, but the protection scope of this disclosure is not limited to this. Any person familiar with the technical field can easily think of various equivalent modifications or substitutions within the technical scope disclosed in this disclosure, and these modifications or substitutions should be covered by the protection scope of this disclosure. Therefore, the protection scope of this disclosure should be subject to the protection scope of claims.

## Claims

1. An electrocardiography acquisition method, which is applied to an electrocardiography acquisition apparatus, **characterized in that**, the method comprises:
receiving a first acquisition instruction; and in response to the first acquisition instruction, obtaining first electrocardiographic data, which is acquired by lead electrode(s) of multiple electrocardiographic leads which are connected with a target object;
receiving an addition instruction; in response to the addition instruction, displaying, on a display interface of the electrocardiography acquisition apparatus, a graphical position corresponding relationship between a first position and a second position of a predetermined electrocardiographic lead in the multiple electrocardiographic leads; or in response to the addition instruction, displaying, on a display interface of the electrocardiography acquisition apparatus, a graphical information entry which is capable of viewing a position corresponding relationship between a first position and a second position, and when the graphical information entry is scanned by an electronic device, displaying the position corresponding relationship on a display interface of the electronic device; wherein the position corresponding relationship is configured to guide a user to move a lead electrode of the predetermined electrocardiographic lead from the first position to the second position; the position corresponding relationship at least comprises a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the first electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, obtaining second electrocardiographic data which is acquired by the lead electrode(s) of the multiple electrocardiographic leads.

2. An electrocardiography acquisition method, which is applied to an electrocardiography acquisition apparatus, **characterized in that**, the method comprises:
determining historical electrocardiographic data of a target object;
receiving an addition instruction; in response to the addition instruction, displaying, on a display interface of the electrocardiography acquisition apparatus, a graphical position corresponding relationship between a first position and a second position of a predetermined electrocardiographic lead in multiple electrocardiographic leads; or in response to the addition instruction, displaying, on a display interface of the electrocardiography acquisition apparatus, a graphical information entry which is capable of viewing a position corresponding relationship between a first position and a second position, and when the graphical information entry is scanned by an electronic device, displaying the position corresponding relationship on a display interface of the electronic device; wherein the historical electrocardiographic data is data acquired by lead electrode(s) of the multiple electrocardiographic leads and obtained by the electrocardiography acquisition apparatus in response to a first acquisition instruction; wherein the position corresponding relationship is configured to guide a user to move a lead electrode of the predetermined electrocardiographic lead from the first position to the second position; the position corresponding relationship at least comprises a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the historical electrocardiographic data; the second position icon is located above the first image element, so as to indicate a position of the target object at which position the lead electrode of the predetermined electrocardiographic lead is to be connected;
receiving a second acquisition instruction; and in response to the second acquisition instruction, obtaining second electrocardiographic data which is acquired by the lead electrode(s) of the multiple electrocardiographic leads.

3. The electrocardiography acquisition method according to any one of claims 1-2, **characterized in that**, the movement guidance information comprises at least one of: movement guidance text, movement guidance path and movement guidance direction; and/or icon attributes of at least some icons in the first position icon and/or in the second position icon are different, wherein the icon attribute comprises at least one of: an icon color, an icon shape, and an icon size; and/or the position corresponding relationship further comprises a first position name which corresponds to the first position and a second position name which corresponds to the second position, wherein the first position name is different from the second position name.

4. The electrocardiography acquisition method according to any one of claims 1-3, **characterized in that**, a mode for displaying the position corresponding relationship comprises at least one of: displaying at least through a static image; displaying at least through a dynamic image, and displaying at least through a video;
preferably, the mode for displaying at least through a static image comprises displaying by combining the static image with at least one of text and voice; and/or
the mode for displaying at least through a dynamic image comprises displaying by combining the dynamic image with at least one of text and voice; and/or
the mode for displaying at least through a video comprises displaying by combining the video with at least one of text and voice.

5. The electrocardiography acquisition method according to any one of claims 1-4, **characterized in that**, the information entry at least comprises a two-dimensional code, which is configured to display the position corresponding relationship between the first position and the second position on the display interface of the electronic device after being scanned by the electronic device.

6. The electrocardiography acquisition method according to any one of claims 1-5, **characterized in that**, further comprising:
outputting a conventional electrocardiogram (ECG) according to the first electrocardiographic data or the historical electrocardiographic data, and/or outputting an additional ECG according to the second electrocardiographic data;
preferably, the conventional ECG comprises a first name which corresponds to the predetermined electrocardiographic lead, the additional ECG comprises a second name which corresponds to the predetermined electrocardiographic lead, wherein the first name and the second name are different; or
the electrocardiography acquisition method further comprises:
displaying the conventional ECG, and switching to display the additional ECG from the conventional ECG, when a first switch instruction is received; or
displaying the additional ECG, and switching to display the conventional ECG from the additional ECG, when a second switch instruction is received.

7. The electrocardiography acquisition method according to any one of claims 1-6, **characterized in that**, the first acquisition instruction is generated by a user through triggering a first electrocardiography acquisition entry, the second acquisition instruction is generated by a user through triggering a second electrocardiography acquisition entry;
the first electrocardiography acquisition entry comprises at least one of: a button which is arranged at the electrocardiography acquisition apparatus, a touch-control element which is arranged at the electrocardiography acquisition apparatus, and a virtual icon which is displayed on the display interface of the electrocardiography acquisition apparatus; the second electrocardiography acquisition entry comprises at least one of: a button which is arranged at the electrocardiography acquisition apparatus, a touch-control element which is arranged at the electrocardiography acquisition apparatus, and a virtual icon which is displayed on the display interface of the electrocardiography acquisition apparatus.

8. The electrocardiography acquisition method according to claim 7, **characterized in that**, the first electrocardiography acquisition entry is a first virtual icon which is displayed on the display interface of the electrocardiography acquisition apparatus, and the second electrocardiography acquisition entry is a second virtual icon which is displayed on the display interface of the electrocardiography acquisition apparatus, wherein the first virtual icon and the second virtual icon are capable of being simultaneously displayed on a same display interface of the electrocardiography acquisition apparatus.

9. The electrocardiography acquisition method according to claim 8, **characterized in that**, the display interface of the electrocardiography acquisition apparatus comprises a real-time electrocardiography interface for displaying real-time electrocardiographic data of the target object, a report preview interface for previewing an electrocardiographic report which is generated according to real-time electrocardiographic data, and a historical electrocardiography interface for displaying historical electrocardiographic data;
wherein, the first virtual icon and the second virtual icon are capable of being simultaneously displayed on at least one of the real-time electrocardiography interface, the report preview interface, and the historical electrocardiography interface.

10. The electrocardiography acquisition method according to any one of claims 1-6, **characterized in that**, the display interface of the electrocardiography acquisition apparatus comprises a real-time electrocardiography interface for displaying real-time electrocardiographic data which is acquired by the lead electrode(s) of the multiple electrocardiographic leads, a report preview interface for previewing an electrocardiographic report which is generated from the real-time electrocardiographic data, and a historical electrocardiography interface for displaying historical electrocardiographic data;
the addition instruction is generated by a user through triggering an addition entry, wherein the addition entry comprises at least one of: a button which is arranged at the electrocardiography acquisition apparatus, a touch-control element which is arranged at the electrocardiography acquisition apparatus, and a virtual icon which is displayed on the display interface of the electrocardiography acquisition apparatus;
wherein, the addition entry is arranged on at least one of the real-time electrocardiography interface, the report preview interface, and the historical electrocardiography interface;
when the addition entry, which is arranged on the report preview interface or the historical electrocardiography interface, is triggered, a data association is established between the second electrocardiographic data and the first electrocardiographic data, or between the second electrocardiographic data and the historical electrocardiographic data, so as to display, in a same electrocardiographic report, an ECG which corresponds to the first electrocardiographic data or the historical electrocardiographic data and an ECG which corresponds to the second electrocardiographic data;
when the addition entry, which is arranged on the real-time electrocardiography interface, is triggered, an ECG which corresponds to the first electrocardiographic data or the historical electrocardiographic data and an ECG which corresponds to the second electrocardiographic data are displayed in different electrocardiographic reports while outputting said electrocardiographic reports.

11. The electrocardiography acquisition method according to any one of claims 1-10, **characterized in that**, further comprising:
in response to the addition instruction, displaying, by the electrocardiography acquisition apparatus, a third position icon which corresponds to a third position of the target object at which position a limb lead in the multiple electrocardiographic leads is connected, wherein the third position icon is configured to indicate a reference connection position between a lead electrode of the limb lead and the target object;
acquiring, by the electrocardiography acquisition apparatus, the first electrocardiographic data or the historical electrocardiographic data of the target object through the lead electrode(s) of the multiple electrocardiographic leads, when the lead electrode of the predetermined electrocardiographic lead is connected at a position of the target object, which position is indicated by the first position, and the lead electrode of the limb lead is connected at the reference connection position;
preferably, the first position icon and the second position icon are configured to simulate the lead electrode of the predetermined electrocardiographic lead; the third position icon is configured to simulate the lead electrode of the limb lead.

12. An electrocardiography acquisition apparatus, **characterized in that**, comprising an electrocardiography acquisition host, and a control apparatus which is in communicative connection with the electrocardiography acquisition host; wherein the control apparatus is configured to:
receive a first acquisition instruction; and in response to the first acquisition instruction, control the electrocardiography acquisition host to obtain first electrocardiographic data, which is acquired by lead electrode(s) of multiple electrocardiographic leads which are connected with a target object;
receive an addition instruction; in response to the addition instruction, control the electrocardiography acquisition host to display a graphical position corresponding relationship between a first position and a second position of a predetermined electrocardiographic lead in the multiple electrocardiographic leads; or in response to the addition instruction, control the electrocardiography acquisition host to display a graphical information entry which is capable of viewing a position corresponding relationship between a first position and a second position, and when the graphical information entry is scanned by an electronic device, display the position corresponding relationship on a display interface of the electronic device; wherein the position corresponding relationship is configured to guide a user to move a lead electrode of the predetermined electrocardiographic lead from the first position to the second position; the position corresponding relationship at least comprises a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the first electrocardiographic data; the second position icon is located above the first image element, so as to indicate a position of the target object at which position the lead electrode of the predetermined electrocardiographic lead is to be connected;
receive a second acquisition instruction; and in response to the second acquisition instruction, control the electrocardiography acquisition host to obtain second electrocardiographic data which is acquired by the lead electrode(s) of the multiple electrocardiographic leads.

13. An electrocardiography acquisition apparatus, **characterized in that**, comprising an electrocardiography acquisition host, and a control apparatus which is in communicative connection with the electrocardiography acquisition host; wherein the control apparatus is configured to:
determine historical electrocardiographic data of a target object;
receive an addition instruction; in response to the addition instruction, control the electrocardiography acquisition host to display a graphical position corresponding relationship between a first position and a second position of a predetermined electrocardiographic lead in multiple electrocardiographic leads; or in response to the addition instruction, control the electrocardiography acquisition host to display a graphical information entry which is capable of viewing a position corresponding relationship between a first position and a second position, and when the graphical information entry is scanned by an electronic device, display the position corresponding relationship on a display interface of the electronic device;
wherein the historical electrocardiographic data is data acquired by lead electrode(s) of the multiple electrocardiographic leads and obtained by the electrocardiography acquisition apparatus in response to a first acquisition instruction; wherein the position corresponding relationship is configured to guide a user to move a lead electrode of the predetermined electrocardiographic lead from the first position to the second position; the position corresponding relationship at least comprises a first image element, a first position icon which corresponds to the first position, a second position icon which corresponds to the second position, and movement guidance information which indicates to move the lead electrode of the predetermined electrocardiographic lead between the first position and the second position; wherein the first image element is configured to simulate the target object and/or region(s) at the target object, which region(s) is(are) connected with the lead electrode(s) of the multiple electrocardiographic leads, and the first position icon is located above the first image element, so as to indicate a position of the target object at which position the lead electrode of the predetermined electrocardiographic lead is connected while acquiring the historical electrocardiographic data; the second position icon is located above the first image element, so as to indicate a position of the target object at which position the lead electrode of the predetermined electrocardiographic lead is to be connected;
receive a second acquisition instruction; and in response to the second acquisition instruction, control the electrocardiography acquisition host to obtain second electrocardiographic data which is acquired by the lead electrode(s) of the multiple electrocardiographic leads.

14. An electrocardiography acquisition method, which is applied to an electrocardiography acquisition apparatus, **characterized in that**, the method comprises:
receiving a first acquisition instruction; and in response to the first acquisition instruction, obtaining first electrocardiographic data, which is acquired by lead electrode(s) of multiple electrocardiographic leads which are connected with a target object;
receiving an addition instruction; in response to the addition instruction, displaying, on a display interface of the electrocardiography acquisition apparatus, a position corresponding relationship between a first position and a second position of a predetermined electrocardiographic lead in the multiple electrocardiographic leads, or an information entry which is capable of viewing a position corresponding relationship between a first position and a second position; wherein the first position is configured to indicate a position of the target object at which position a lead electrode of the predetermined electrocardiographic lead is connected while acquiring the first electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position;
receiving a second acquisition instruction; and in response to the second acquisition instruction, obtaining second electrocardiographic data which is acquired by the lead electrode(s) of the multiple electrocardiographic leads.

15. An electrocardiography acquisition method, which is applied to an electrocardiography acquisition apparatus, **characterized in that**, the method comprises:
determining historical electrocardiographic data of a target object;
receiving an addition instruction; in response to the addition instruction, displaying, on a display interface of the electrocardiography acquisition apparatus, a position corresponding relationship between a first position and a second position of a predetermined electrocardiographic lead in multiple electrocardiographic leads, or an information entry which is capable of viewing a position corresponding relationship between a first position and a second position; wherein the historical electrocardiographic data is data acquired by lead electrode(s) of the multiple electrocardiographic leads and obtained by the electrocardiography acquisition apparatus in response to a first acquisition instruction; wherein the first position is configured to indicate a position of the target object at which position a lead electrode of the predetermined electrocardiographic lead is connected while acquiring the historical electrocardiographic data; the second position is configured to indicate a position of the target object at which position the lead electrode of the predetermined electrocardiographic lead is to be connected; the position corresponding relationship is configured to guide a user to move a lead electrode of the predetermined electrocardiographic lead from the first position to the second position; and
receiving a second acquisition instruction; and in response to the second acquisition instruction, obtaining second electrocardiographic data which is acquired by the lead electrode(s) of the multiple electrocardiographic leads.
